(19) Europäisches Patentamt
European Patent Office
Office européen des brevets

(11) Publication number: **0 093 915 A1**

# (12) EUROPEAN PATENT APPLICATION

(21) Application number: **83103933.4**

(22) Date of filing: **22.04.83**

(51) Int. Cl.³: **C 07 D 487/04**, C 07 D 519/00, A 61 K 31/40 // C07D205/08, C07D277/42, C07D277/46, C07D213/74, C07D213/75, C07D261/14, C07D233/88 ,(C07D487/04, 209/00, 205/00),(C07D519/00, 487/00, 471/00),(C07D519/00, 487/00, 487/00),(C07D519/00, 498/00, 487/00)

(30) Priority: **10.05.82 GB 8213456**

(43) Date of publication of application: **16.11.83 Bulletin 83/46**

(84) Designated Contracting States: **AT BE CH DE FR GB IT LI LU NL SE**

(71) Applicant: **GRUPPO LEPETIT S.P.A., 8, Via Roberto Lepetit, I-20124 Milano (IT)**

(72) Inventor: **Omodei-Sale, Amedeo, 57, Via Papa Giovanni XXIII, Voghera (PV) (IT)**
Inventor: **Favara, Duccio, 5, Via Ripamonti, Como (IT)**
Inventor: **Consonni, Pietro, 18, Via Angera, Milano (IT)**
Inventor: **Cecchi, Roberto, 1, Viale Dei Tigli, Lodi, (MI) (IT)**

(54) **Novel beta-lactam derivatives.**

(57) New β-lactam antibiotics of the general formula

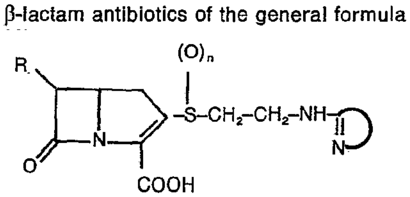

wherein R is $(C_1-C_6)$alkyl or $(C_3-C_6)$alkenyl, n is zero or one and the stylized group 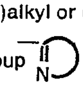 represents a mono- or polycyclic N-containing heterocyclic group, and their pharmaceutically acceptable salts and esters. Also described and claimed are the process for preparing the new compounds and the pharmaceutical compositions containing them.

EP 0 093 915 A1

NOVEL β-LACTAM DERIVATIVES

The present invention relates to a novel class of β-lactam antibiotics of the general formula

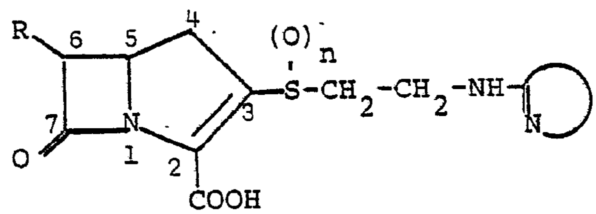

I

wherein R is $(C_1-C_6)$alkyl or $(C_3-C_6)$alkenyl,

n is zero or one and

the stylized group 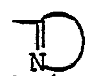 represents a mono- or polycyclic N-containing heterocyclic group,

and their pharmaceutically acceptable salts and esters. This invention also relates to the process for preparing such compounds, to pharmaceutical compositions containing them and to methods of treatment comprising administering such compounds and compositions when an antibiotic effect is desired.

In the present specification and claims, the term "$(C_1-C_6)$alkyl" is intended to refer to a straight or branched alkyl group containing from 1 to 6 carbon atoms, and the term "$(C_3-C_6)$alkenyl" designates a straight or branched alkenyl group containing from 3 to 6 carbon atoms and one or two double bonds. The term "mono- or polycyclic N-containing heterocyclic group" identifies mono-, bi-, and tri-cyclic groups, each cycle consisting of 5- or 6-atoms, which may optionally contain 1,2, or 3 additional heteroatoms, each independently selected from N, O, or S, and the carbon and nitrogen atoms of any

such ring may carry substituents selected from the group consisting of hydroxy, halogen, amino, mono- or di-$(C_1-C_6)$alkylamino, nitro, $(C_1-C_6)$alkoxy, substituted or unsubstituted $(C_1-C_6)$alkyl, substituted or unsubstituted $(C_3-C_6)$alkenyl, phenyl, substituted phenyl, substituted or unsubstituted phenyl-$(C_1-C_6)$-alkyl, and substituted or unsubstituted 5- or 6-membered heterocyclic, wherein the substituent or substituents are selected from halogen, hydroxy, alkoxy, carboxy, alkyl, or phenyl.

A preferred group of compounds of the present invention comprises those compounds of formula I wherein R is ethyl and n and the stylized group 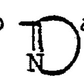 are as defined above, and their corresponding pharmaceutically acceptable salts and esters.

A most preferred group of compounds comprises those compounds of formula I wherein R is ethyl, n is zero and the stylized group 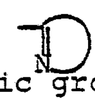 represents a monocyclic N-containing heterocyclic group, and their corresponding pharmaceutically acceptable salts and esters.

For the purposes of the present application, the term "pharmaceutically acceptable salts" include those salts of the alkali and alkaline earth metals, of these the sodium and potassium salts are preferred. The term "pharmaceutically acceptable esters" refers to the esters which are known in the bicyclic β-lactam antibiotic art, such as the cephalosporins and penicillins, to be effective as pharmaceutically acceptable esters. Among the suitable esters there are those esters which derive from the carboxylic acids I by replacing the carboxyl group with a

$-COOR_1$ group wherein $R_1$ stands for one of the following groups:
straight or branched alkyl having from 1 to 6 carbon atoms, such as methyl, ethyl, t-butyl and the like, alkanoyloxyalkyl wherein the alkanoyloxy portion is straight or branched and has 1 to 6 carbon atoms and the alkyl portion has 1 to 6 carbon atoms, such as pivaloyloxymethyl; halo-alkyl wherein halo is chloro and the alkyl portion is straight or branched having 1 to 6 carbon atoms, e.g. 2,2,2-trichloroethyl; aralkyl and lower alkoxy-and nitro- substituted aralkyl, such as benzyl, benzhydryl, trityl, o-nitrobenzyl, p-nitrobenzyl, p-methoxybenzyl; phthalidyl, dimethyl-phthalidyl, benzyloxy-alkyl having 8 to 10 carbon atoms such as benzyloxymethyl and benzyloxyethyl, and p-nitro-benzyloxy-methyl. The compounds of the present invention may have the cis- or trans- geometry about the β-lactam, that is to say that the hydrogen atoms at C-5 and C-6 may lie cis or trans to each other.
Formula I above therefore encompasses either mixtures of the optically active trans or cis isomers or the single optically active components. In most cases however the antibacterial activity of the end carbapenem antibiotics and their inhibitory activity against β-lactamases is tied to the trans configuration; therefore a preferred group of compounds of formula I includes those compounds in which the hydrogen atoms at C-5 and C-6 have trans stereochemistry.

The compounds of the present invention can be easily prepared by the process which is schematically represented in the following Chart I

CHART I

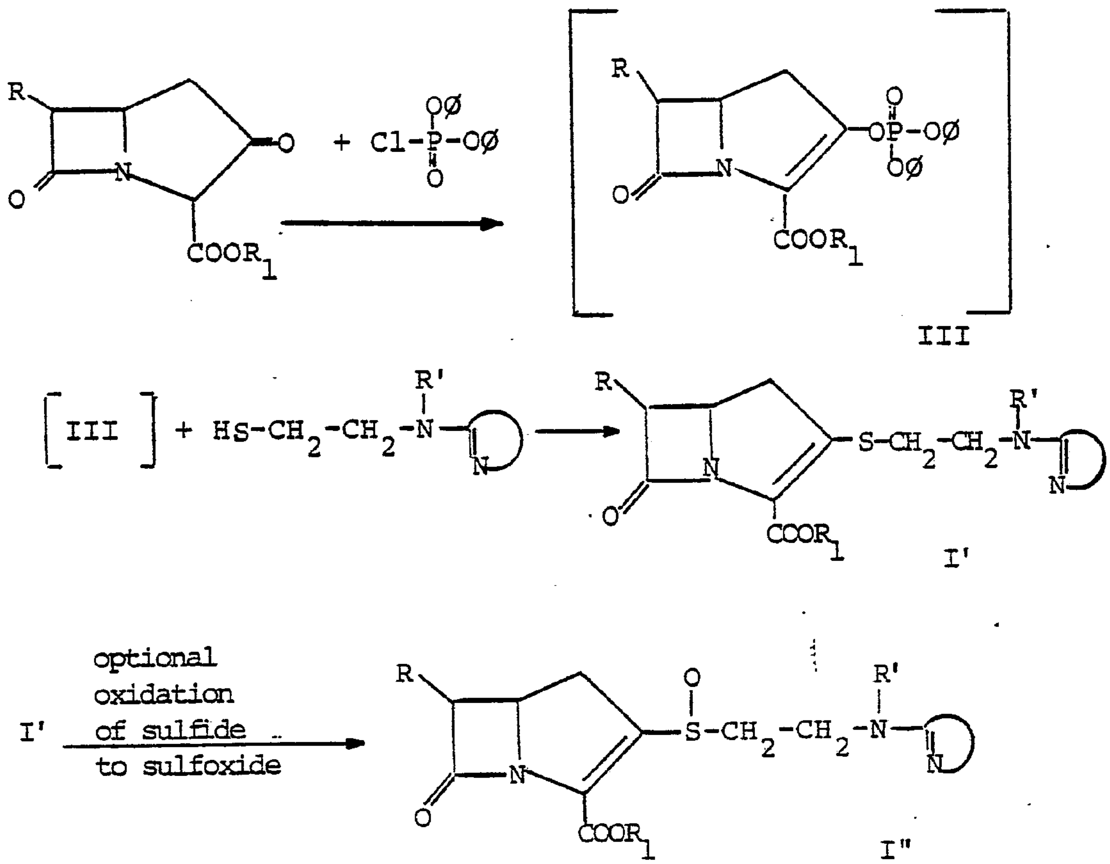


III

I'

I"

I' or I" catalytic deprotection I

In the first step of the process described in Chart I, the bicyclic ketoester of formula II, wherein R is as defined above and the $-COOR_1$ group represents a cleavable ester group or a pharmaceutically acceptable ester, is converted into the enol-phosphate III, by reaction with diphenyl chlorophosphate. The obtained intermediate, without isolation, is reacted with a suitably selected nucleophile of formula $HS-CH_2-CH_2-\overset{R'}{N}-$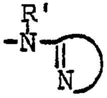, wherein the stylized group 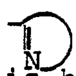 has the same meanings as before, and R' is hydrogen or an easily removable protecting group of the amino funtion. In some particular cases in fact, the heterocyclic substituent may enhance the nucleophylicity of the amino group and in order to avoid the competitive attack of the amino group instead of the thiol group on the enol-phosphate III, it is necessary to protect the amino group during the course of the reaction. This protective group, typically a benzyl-or p-nitrobenzylester group, may then be easily removed by hydrogenolysis. The reaction between the enol phosphate III and the nucleophile $HS-CH_2-CH_2-\overset{R'}{N}-$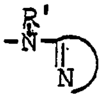 affords the ester I'.

If desired the sulfide group can then be oxidized to sulfoxide to give the corresponding esters I".

When a compound of formula I in the form of the free acid or as a corresponding salt thereof is desired, it is obtained, according to the last step described in Chart I above, by catalytic deprotection of the corresponding ester of formula I' or II". Of course in this case, in the starting $\beta$-ketoester II, $R_1$ must be selected properly within the class of readily removable protecting groups.

Suitable cleavable esters are for instance the benzyl, p-nitrobenzyl, benzhydryl, trytyl, t-butyl and trichloroethyl esters and the like.

In particular the first step reported in Chart I above is accomplished by contacting a solution of the bicyclic ketoester II in a solvent such as acetonitrile, dimethyl-formamide, methylene chloride, or the like solvents with the stoichiometric amount or a slight excess of diphenyl chlorophosphate in the presence of a tertiary nitrogen base such as ethyldiisopropylamine, triethylamine or the like, to neutralize the hydrochloric acid produced during the course of the reaction.

The reaction is preferably carried out at low temperatures, most preferably at a temperature comprised between about -10°C and about 0°C.
In the second step of the reaction, a stoichiometric to four-fold excess of the suitably selected nucleophile HS-$CH_2$-$CH_2$-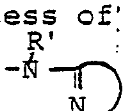 , is added to the reaction mixture containing the intermediate III. The reaction which is typically carried out at a temperature comprised between -30°C and + 20°C, and preferably between -20°C and 10°C, is accomplished in 20 minutes to 4 hours. In general the ester I' thus obtained crystallizes out from the mixture during the course of the reaction and is recovered by filtration. In some cases however, it may be necessary to concentrate the reaction mixture to a small volume and to chill it in order to complete the precipitation.

The S-oxidation of the compounds I' to the sulfoxides I", described in the third step, can be carried out by the common methods frequently used in the β-lactam antibiotic art. For example, the ester I' is reacted with a mild oxidizing agent which does not substantially act on the β-lactam skeleton such as organic peracids, especially perbenzoic acid and m-chloroperbenzoic acid, phenyldichloroiodide or sodium metaperiodate. Substituted perbenzoic acids, and in particular m-chloroperbenzoic acid, are preferred. Typically, the oxidation reaction is carried out in the presence of an inert organic solvent, such as lower halogenated hydrocarbons, and preferably methylene chloride or chloroform, at a low temperature, preferably comprised between -30°C and 0°C, using a stoichiometric to a 10% molar excess of the oxidizing agent to the ester I'. The resulting sulfoxides are then isolated and purified by the usual methods known <u>per se</u> to any skilled chemist.

When a compound of formula I is desired in the form of free acid, the $R_1$ group is splitted off in many instances by hydrogenolysis. Said reaction can be performed by hydrogenating the corresponding esters of formula I' or I" in a mixture of an inert organic solvent such as dioxane, tetrahydrofuran and the like inert organic solvents non susceptible of being hydrogenated, and a buffer having pH comprised between 6 and 9 under a hydrogen pressure of from 1 to about 4 atmospheres in the presence of a hydrogenation catalyst such as for instance palladium on charcoal, platinum oxide and the like.

The resulting compound of formula I is then isolated and purified by the methods which are generally used in related art in isolating and purifying carboxylic acid-type antibiotics.

These methods are well known to any skilled chemist and there are no critical procedures to be followed in the recovery and purification of the obtained compounds. Anyway, for purposes of demonstration only, a set of standard representative operations illustrating a convenient way for isolating and purifying the end acids I is recited herein below. After having removed the catalyst by filtration, the organic solvent is evaporated off and the aqueous solution is washed with an organic water-immiscible solvent, purified by ion exhange chromatography on QAE-Sephadex A-25 eluting with a gradient of phosphate buffers pH 8 and 1.5% KCl monitoring the fractions by HPLC, and desalted through a column of Amberlite® XAD-2 resin. The appropriate fractions are combined, evaporated to small volume, filtered on Millipore (R), and lyophilized to give the desired compounds in the form of their alkali metal salts. Potassium salts are generally preferred, since they appeared to be less hygroscopic than the corresponding sodium salts.

The starting β-ketoester of formula II are prepared from the corresponding azetidinyl ethanols of formula IV

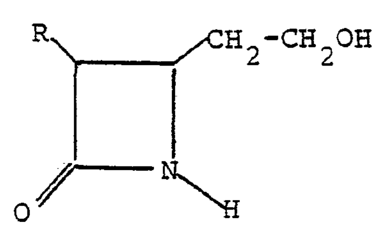

IV

wherein R is as defined above,

through the multi-step process summarized in following Chart II

CHART II

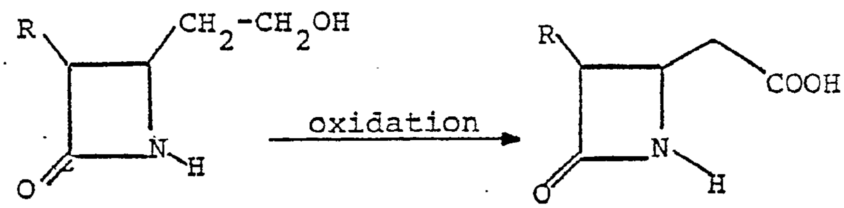


IV

V

V

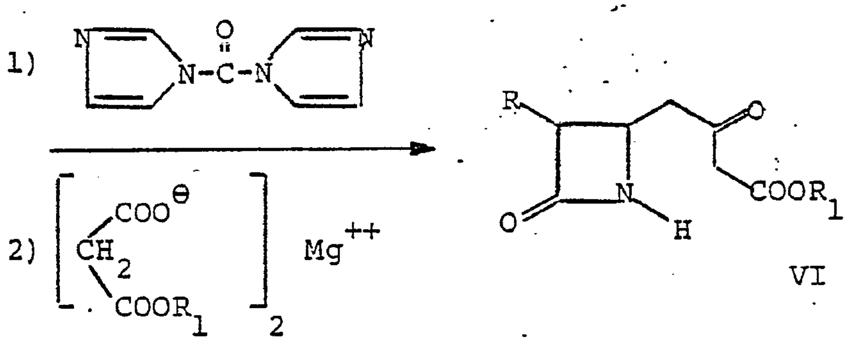


VI

VI +

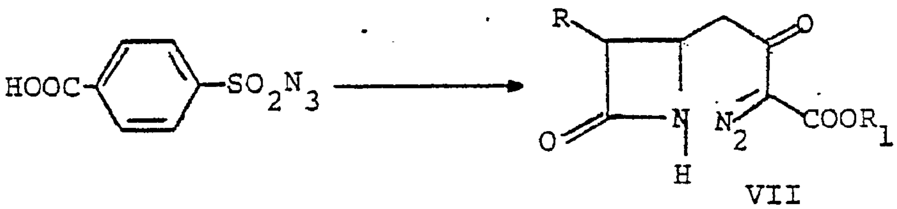

VII

VII

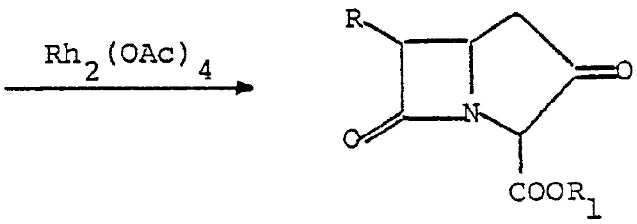


II

For the oxidation of the ethanol (IV) to the corresponding acid V, several different oxidizers may be employed such as for instance oxygen in the presence of a catalyst such as platinum adsorbed on carbon, the Jones reagent and permanganate; however the use of aqueous potassium permanganate buffered at a pH between 4 and 9, and preferably between 6.6 and 8.5, at room temperature, is preferred.
The amount of oxidizing agent used in this step can be varied widely depending on the type of oxidizing agent and the reaction conditions employed.
When according to a preferred embodiment, aqueous potassium permangate is employed, the suitable amount is generally comprised between 0.7 and 1.8 mole of permanganate per mole of alkanol of formula IV. Under these conditions the reaction is generally complete in 3 to 15 hours.

When the oxidation reaction, which may be monitored by G.L.C. analysis, is complete, the excess of unreacted oxidizing agent remaining in the reaction mixture is decomposed; if necessary, the corresponding reduced species or the catalyst, if any, is removed, and the resulting acid of formula V is isolated and purified by methods known per se. In the second step, the side chain of the acid is elongated by transforming it into the imidazolyl derivative and then, without isolation of the obtained intermediate, reacting it with the magnesium salt of a malonic acid mono ester of formula $\left[\begin{matrix} COO^{\ominus} \\ CH_2\text{-}COOR_1 \end{matrix}\right] \frac{1}{2} Mg^{++}$.

The reaction easily proceeds in high yield at room temperature in a polar aprotic organic solvent such as tetrahydrofuran dioxane, dimethyl sulfoxide and the like. The carbonyldiimidazole reagent is preferably employed in a slight excess over the stoichiometric, while a two to four-fold molar ratio of the malonic mono-ester mono-magnesium salt to the starting acid of formula V is preferably employed.

The obtained β-keto ester of formula VI is recovered from the reaction mixture by acidification and extraction with a suitable organic solvent which is then boiled off and it is submitted, as such, to the diazo-transfer reaction described in the third step of Chart II. This reaction is carried out by contacting an equimolar proportion or a slight excess of p-carboxybenzenesulfonylazide and the β-keto ester of formula VI in the presence of an inert organic solvent such as acetonitrile, dimethylformamide, methylene chloride and the like, and of a tertiary organic nitrogen base.

The temperature of the reaction is generally comprised between -10°C and 30°C and preferably between 5°C and room temperature. Finally, decomposition of the ester VII with a catalytic amount of $Rh_2(OAc)_4$, by heating the catalyst first and then slowly adding the diazo (VII) solution thereto, gives the bicyclic keto ester (II) in almost quantitative yield. The desired keto ester II is isolated and, if desired, purified, by conventional procedures readily apparent to any skilled chemist. In their turn, the azetidin-ethanol derivatives of formula IV, are prepared by either of the two different methods described in our copending European Patent Application No.82101463.6 filed on February 26,1982

having the title:"New β-lactam acetic acid derivatives, the process for preparing them, their use as intermediates for 1-azabicyclo[3.2.0]hept-2-ene antibiotics", which are incorporated herein by reference. Briefly, the two methods are schematically reported in the following Charts III and IV.

CHART III

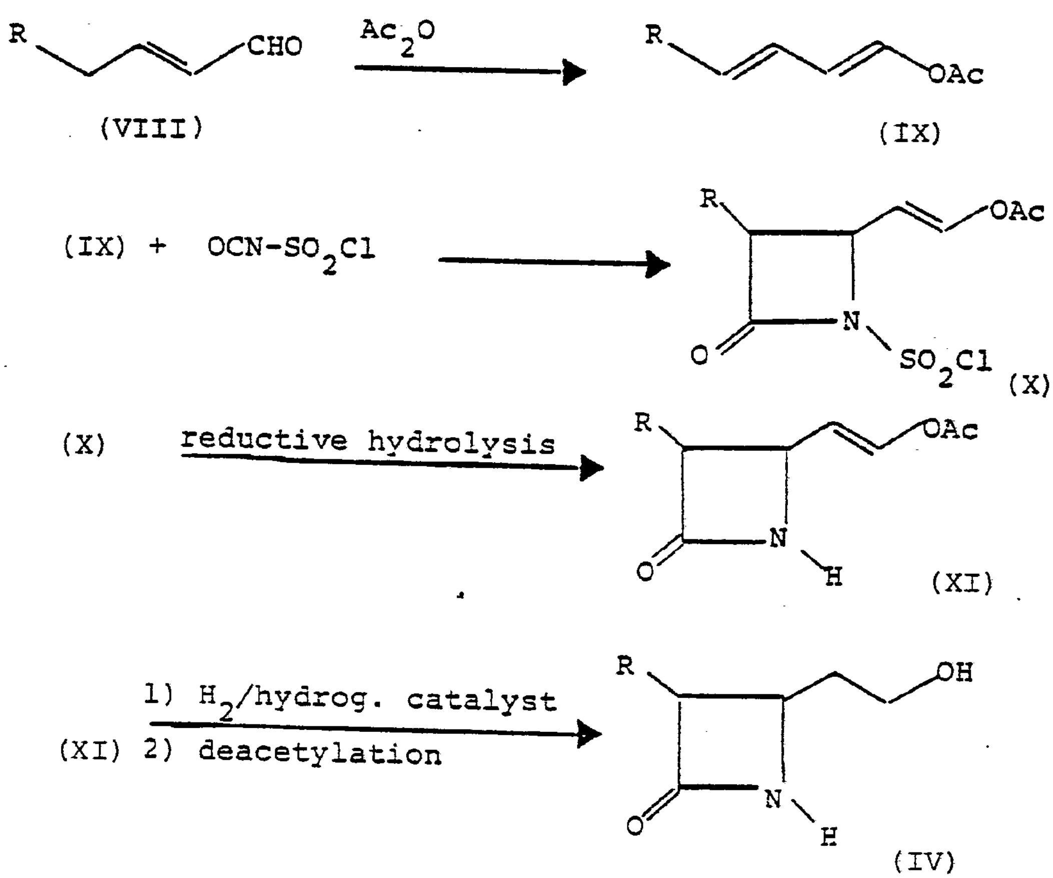

CHART IV

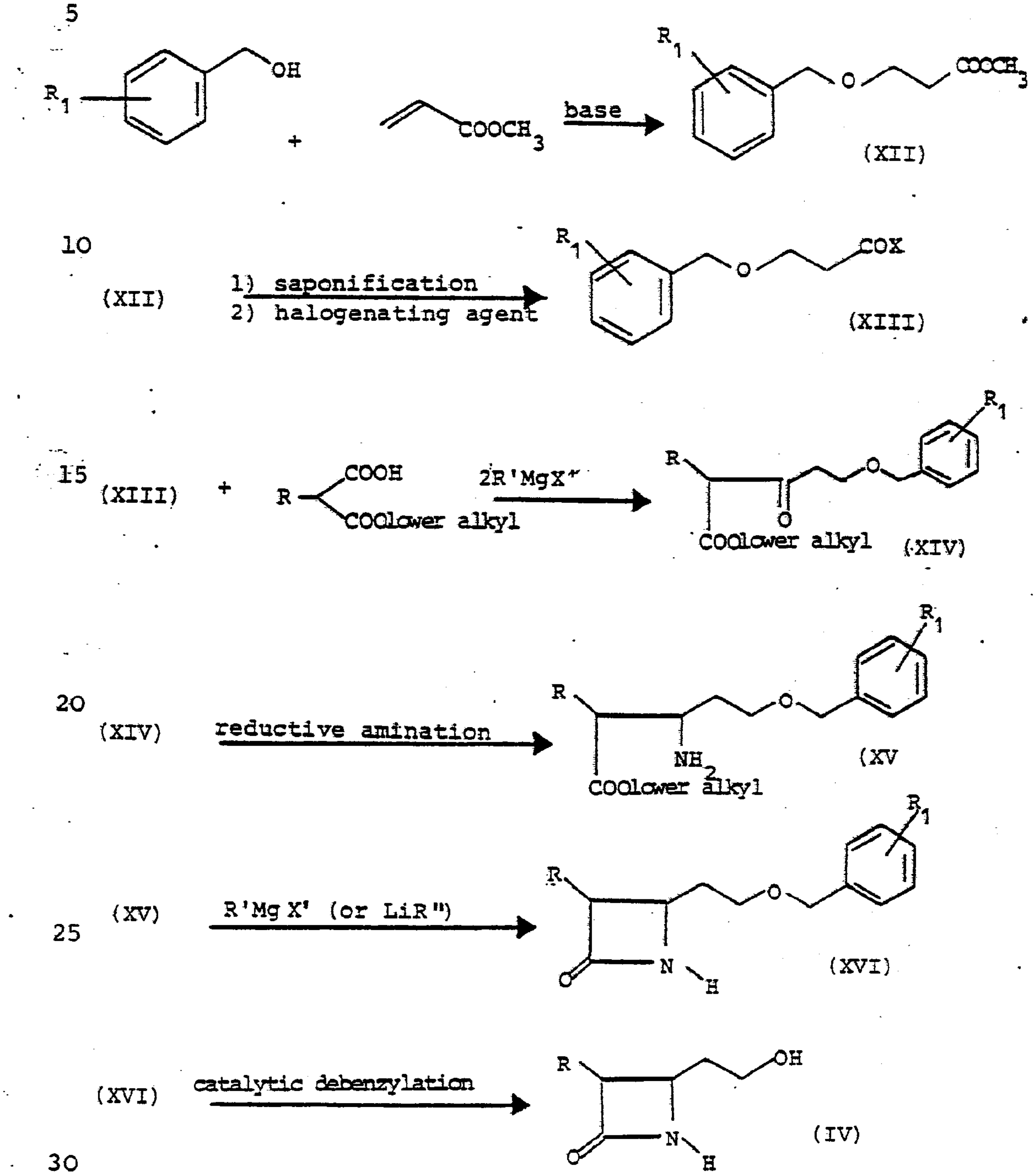


(XII) (XIII) (XIV) (XV) (XVI) (IV)

According to Chart III, the enol acetate of a trans-4-substituted-2-buten-1-al is reacted with chlorosulfonyl isocyanate to give the 2-azetidone (X) which is then submitted to reductive hydrolysis affording the 2-azetidinone (XI) in very high yields. The azetidinone (XI) is then submitted to hydrogenation followed by deacetylation yielding the desired azetidinyl ethanol (IV). More particularly, the formation of the enol-acetate (IX) is achieved by reacting the aldehyde (VIII) with acetic anhydride in the presence of an organic or inorganic mild base such as a tertiary amine, or an alkali metal carbonate, and optionally, but not preferably, in the presence of an inert organic solvent. The reaction proceeds smoothly at room temperature, however sometimes it may be useful to heat the reaction mixture in order to speed up the reaction.

Alternatively, the enol-acetate (IX) may be prepared by reacting the trans-4-substituted-2-buten-1-al with isopropenyl acetate in the presence of catalytic amounts of p-toluenesulfonic acid and cupric acetate.

The second step, i.e. the [2+2] cyclo-addition of chlorosulfonyl isocyanate to the enol-acetate, is carried out in an inert organic solvent such as lower alkyl ethers, aliphatic and aromatic hydrocarbons, halogenated hydrocarbons, nitromethane, acetonitrile and the like, under inert atmosphere and keeping the temperature in the range of -20°C-5°C. This reaction may either be carried out in batch or, more advantageously, as a continuous process. The organic solution containing the chlorosulfonyl derivative is submitted to reductive hydrolysis conditions

by treatment with water buffered at a pH of about 6-8 containing a water soluble reducing agent such as sodium sulfite.

The hydrogenation of (XI) may be carried out in the presence of a hydrogenation catalyst such as for instance Platinum or Palladium, preferably adsorbed on a carbon or asbestos inert carrier, or Nickel-Raney, at a temperature of from 20 to 60°C, under a pressure ranging from the normal pressure to about 20 atm. Preferably, the reduction is carried out at room temperature and under a pressure of about 3 atmospheres using a palladium on carbon catalyst. Solvents which may suitably be employed in this step are those commonly employed in hydrogenation reactions such as for instance ethyl acetate, dioxane, tetrahydrofuran, alcohols and the like. Finally, desacetylation to the alcohol (IV) occurs with very high yields by treatment with methanol containing a base such as sodium methoxide or potassium carbonate or cyanide.

The six step synthesis reported in Chart IV begins with the base-catalyzed addition of benzyl or substituted benzyl alcohol to methyl acrylate to give the 3-benzyloxy-propionic acid methyl ester (XII) wherein $R_1$ may represent hydrogen, lower alkyl, lower alkoxy, or nitro. The addition is carried out in the presence of an inert organic solvent which does not interfere with the reaction course, such as for instance benzene, toluene, xylene, and the like, using an alkali metal lower alkoxide or hydride or a tri-lower alkyl phosphine as the basic catalyst. Once the addition reaction is completed,

saponification of the ester (XII) with aqueous sodium or potassium hydroxide may be carried out directly on the reaction mixture deriving from the addition reaction, without separation of the ester (XII) being required. 3-Benzyloxypropionic acid which forms is easily recovered from the reaction mixture by means of conventional procedures which involve acidification of the aqueous phase and extraction with a suitable organic solvent which is then boiled off. Reaction of the obtained 3--benzyloxypropionic acid with a halogenating agent, typically thionyl chloride, in the presence of a catalytic amount of dimethylformamide then affords the 3-benzyloxypropanoyl halide (XIII) wherein $R_1$ is as defined above and X is chlorine or bromine.
Condensation of the 3-benzyloxypropanoyl halide (XIII) with the magnesium salt of a malonic acid monolower alkyl ester derivative, obtained by treating a malonic acid monolower alkyl ester derivative $R-<^{COOH}_{COO-\text{lower alkyl}}$ with a lower alkyl magnesium halide, yields the keto-ester (XIV). The formation of the magnesium salt is achieved by contacting the malonic acid monolower alkyl ester derivative with the Grignard reagent R'MgX' wherein R' is a lower alkyl group and X' is chlorine, bromine or iodine in the presence of an inert organic solvent selected from those commonly employed in the Grignard reactions.
Once the salt is formed, 3-benzyloxypropanoyl halide is gradually added while keeping the temperature between about $10^{o}C$ and the room value.

By pouring the reaction mixture in aqueous mineral acids, extracting with an organic solvent immiscible with water, and evaporating off the extracting solvent, the keto-ester (XIV) is recovered. If desired, the raw product (XIV) thus obtained may be purified by means of the usual techniques or it can be used as such in the reductive amination step that leads to the amine derivative (XV).

In this reductive amination, the carbonyl compound (XIV) is treated with a large excess of ammonia, optionally in the form of its acetate, in the presence of a suitable reducing agent.

Sodium cyanoborohydride is a particularly preferred reducing agent, however other reducing agents can be used instead of sodium cyanoborohydride, among them hydrogen and a hydrogenation catalyst and sodium borohydride. The reaction which proceeds smoothly at room temperature and in alcoholic solvents, is preferably carried out at a pH of about 4.5-6.0 for instance by addition of glacial acetic acid.

Acidification of the reaction mixture with concentrated hydrochloric acid, filtration of the ammonium chloride which separates, and concentration of the filtrate to dryness affords a crude residue from which the compound (XV) may be recovered by conventional purification procedures which are entirely familiar to any skilled chemist.

Conversion of the amine derivative (XV) to the β-lactam (XVI) is achieved by treating the amine (XV) with at least two equivalents, and preferably three equivalents,

of a Grignard reagent, R'MgX', wherein R' is a lower alkyl group and X' is chlorine, bromine or iodine, or a lithium reagent R"-Li wherein R" stands for an alkyl, alkenyl or aryl group. The reaction is carried out in an inert, organic solvent such as for instance ethyl ether, tetrahydrofuran, benzene, toluene, xylene and the like, and generally takes from 2 to 4 hours to be completed. The reactants are brought into contact at low temperature, preferably comprised between -30 and + 5°C, and most preferably between -5 and + 5°C, then the reaction proceeds at room temperature. Once the reaction is complete, diluted mineral acid is added at low temperature to give a final acid pH, preferably a final pH of about 3, and the β-lactam (XVI) is extracted with a suitable organic solvent immiscible with water.

The compound (XVI) which is recovered by evaporating off the extracting solvent may then be purified by conventional procedures such as fractional distillation or chromatography or both techniques.

Finally catalytic debenzylation of the β-lactam (XVI), carried out according to the usual procedures known in chemistry, gives the alcohol (IV). Preferably deprotection is achieved by using hydrogen and a hydrogenation catalyst, such as palladium adsorbed on carbon, in acetic acid and in the presence of a catalytic amount of a strong acid such as trifluoroacetic, sulfuric or hydrochloric acid.

All the reaction steps outlined in Charts I and II proceed with retention of the configuration of the carbon atoms

numbered 5 and 6 in the β-lactam structure

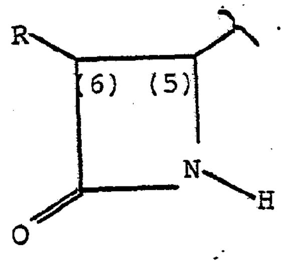

Thus, when starting from the *trans* or *cis* racemates of the alcohol (IV) or acid (V), the *trans* or *cis*, racemates of the end product I are obtained, and when starting from a single *trans* or *cis* enantiomer of the alcohol (IV) or acid (V), one of the *trans* or *cis* enantiomer I is obtained wherein the hydrogen atoms at the 5- and 6- positions have the same absolute configuration of the carbon atoms indicated with (5) and (6) in the starting alcohol or acid. Separation of the *cis*/*trans* mixture into the couples of *cis* and *trans* isomers is generally carried out at the β-lactam enol-acetate (XI) stage, when the process described in Chart III is employed for preparing the alcohol (IV), or at the acid (V) stage when the couples of *cis* and *trans* isomers are separated by crystallization from ethyl acetate. Moreover, when a single *cis* or *trans* enantiomer is desired, the *cis* or *trans* racemate of the acid (V) thus obtained, is then separated into the single optically active components by means of conventional procedures known in chemistry to this purposes, which involve reaction with an optically active base, separation of the diastereoisomeric salts which form by fractional crystallization or preparative HPLC, and separate restoration of the free acids.

Among the optically active bases which may suitably be employed in this separation, there are oxyphene, dehydroabiethylamine, brucine, rosine, cinconine, cinconidine, quinine, ephedrine and the like. Alternativelly, "differential absorption" techniques, using column packed with chiral absorbents, may suitably be employed in this separation.

The reaction partners of the starting $\beta$-ketoester, i.e., the cysteamine derivatives of formula

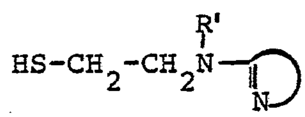

wherein R' is hydrogen

which are generally novel compounds, can be easily prepared by the method outlined in the following Chart V

CHART V

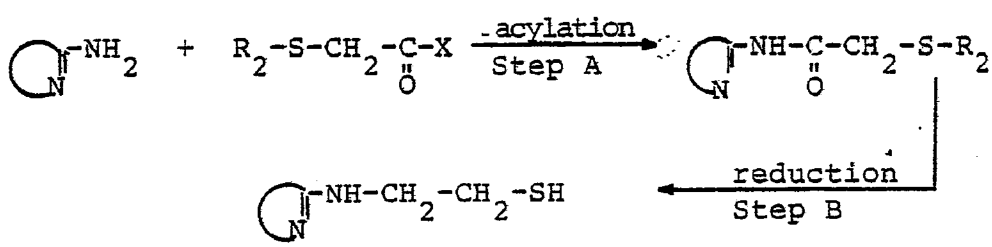


Briefly the above method consists in acylating an $\alpha$-amino heterocyclic compound with a suitable acylating agent of formula $R_2\text{-}S\text{-}CH_2\text{-}\underset{O}{\underset{\|}{C}}\text{-}X$ wherein $R_2$ is a protecting group of the thiol function (which can be easily removed under the conditions employed in step B for the reduction of the amidic carbonyl group) typically a $CH_3\text{-}\underset{O}{\underset{\|}{C}}\text{-}$ or a $HO\text{-}\underset{O}{\underset{\|}{C}}\text{-}CH_2\text{-}S\text{-}$ group, and X represents

a chlorine or bromine atom or a hydroxy group, and then reducing the amidic carbonyl group and simultaneously splitting off the $R_2$ blocking group. As for the acylation step, the reaction is carried out in the presence of a polar aprotic organic solvent such as methylene chloride, chloroform, dimethylsulfoxide, tetrahydrofuran and the like solvents, using equimolar amounts of the two reactants or a slight excess of the α-aminoheterocyclic compound.

When X represents a hydroxy group, the reaction is made to proceed in good yields at room temperature by the use of coupling agents such as dicyclohexylcarbodiimide , N,N'-carbonyldiimidazole and the like agents, and preferably dicyclohexylcarbodiimide.

When an acyl halide is employed (X is chlorine or bromine), the reaction is carried out in the presence of a hydrogen halide acceptor agent to block the hydrohalic acid which forms during the reaction. Among the suitable hydrogen halide acceptor, tertiary organic nitrogen bases, such as triethylamine, diisopropylethyl amine, pyridine, picoline and the like, are preferred.

When an acyl halide is employed, owing to the higher reactivity of this species, the reaction condition must be more carefully controlled. In particular, when using an acyl halide, the temperature of the reaction is generally kept between about $-10^{\circ}C$ and $+ 10^{\circ}C$ and preferably in the range $0-5^{\circ}C$.

The intermediate substituted mercaptoacetylamino-heterocycle is then recovered by the usual procedures and converted into the desired ethanethiol derivative through reduction of the amidic carbonyl group and of the thiol-

protecting group.

The reductive step is carried out with $LiAlH_4$ or by catalytic hydrogenation, and preferably with $LiAlH_4$ because high temperatures and pressure are generally required for the catalytic hydrogenation.

In particular, reduction with $LiAlH_4$ is easily carried out by mixing the intermediate substituted mercapto-acetylamino-heterocycle dissolved in an anhydrous inert organic solvent, typically tetrahydrofuran, with a 3 fold molar amount and preferably a slight excess over the 3-fold amount of the reducing agent suspended in the same solvent. The reaction which smoothly proceeds at temperatures which are initially low, generally comprised between -10°C and + 5°C, and then increase to room temperature, takes from 1 $\frac{1}{2}$ to 3 hours to be complete. The excess of reducing agent is then decomposed by the addition of diluted mineral acids and the obtained cysteamine derivative is recovered from the reaction mixture by the usual methods which are entirely familiar to the skilled chemist.

When for the reasons seen above, it is necessary to protect the amino group, the cysteamine derivative obtained according to the reaction scheme reported in Chart V, is reacted with at least a double molar amount of a suitable protective agent to give the corresponding compound wherein both the mercapto and the amino groups are protected. Then the free thiol group is restored by selective removal of the mercapto-protecting-group with ammonia in methanol.

Analogously, when in addition or alternatively to the amino group, other nucleophilic groups present in the molecule must be protected, the same procedure can be followed

but using the proper molar amount of the protective agent.

The compounds of formula I according to the present invention as well as their pharmaceutically acceptable salts and esters have valuable antibiotic activity against various gram-positive and gram-negative bacteria. The antibacterial spectrum *in vitro* of some representative compounds of the present invention is reported in Table I below.

TABLE I

MIC (µg/ml)

| ORGANISM | 21 | 22 | 23 | 24 | 25 | 26 | 27 |
|---|---|---|---|---|---|---|---|
| S. aureus ATCC 6538 | 0.012 | 0.1 | 0.1 | 0.1 | n.d. | 0.1 | 1.6 |
| S. aureus Smith | 0.05-0.2 | 01-0.8 | 0.1 | 0.4 | 0.1 | 0.1 | 3.1 |
| S. pyogenes c 203 SKF 13400 | n.d. | n.d. | 0.05 | 0.1 | 0.025 | 0.05 | 0.8 |
| S. pyogenes c 203 ISM | 0.012 | 0.1 | n.d. | n.d. | n.d. | n.d. | n.d. |
| S. pneumoniae UC 41 | 0.006 | 0.05 | 0.05 | 0.1 | 0.025 | 0.05 | 1.6 |
| P. vulgaris ATCC 881 | 3.12 | 3.12 | 3.1 | 6.2 | 6.2 | 6.2 | 25 |
| E. coli SKF 12140 | 1.6 | 25 | 6.2 | 12.5 | 6.2 | 6.2 | 6.2 |
| E. cloacae 45 | 1.6 | 25 | 3.1 | 6.2 | 3.1 | 3.1 | 25 |
| E cloacae P 99 1321 E | 3.12 | 25 | 3.1 | 12.5 | 6.2 | 12.5 | 12.5 |
| K. aerogenes 1522 E | 1.6 | 12.5 | 3.1 | 6.2 | 6.2 | 12.5 | 6.2 |

n.d. = not determined

MICs (minimum inhibitory concentrations) were determined in "microtiter" using Brain-heart infusion broth (Difco) or Penassay broth (Difco) for gram-positive or gram-negative bacteria, respectively. *S. pyogenes* and *S. pneumoniae* were tested in Todd-Merwitt broth (Difco). The inoculum was overnight cultures diluted to obtain $10^3$ or $10^2$ CFU/ml respectively in the case of gram-positive or gram-negative strains.
The MIC was defined as the lowest concentration of antibiotic which presented visible growth after overnight incubation at 37°C.

The compounds of the present invention can effectively be employed as the active ingredients of antimicrobial preparations used in human and veterinary medicine for the prevention and treatment of infectious diseases caused by gram-positive and gram-negative bacteria which are susceptible to the active ingredient.

The compounds of the present invention can be administered orally, topically or parenterally. Depending on the route of administration, these compounds can be formulated into various dosage forms. Preparations for oral administration may be in the form of capsules, tablets, liquid solutions or suspension. As known in the art the capsules and tablets may contain, in addition to the active ingredient, conventional excipients such as diluents, e.g. lactose, calcium phosphate, sorbitol and the like, lubricants, e.g. magnesium stearate, talc, polyethylene glycol, binding agents, e.g. polyvinylpyrrolidone, gelatin, sorbitol, tragacanth, acacia, flavouring agents, and acceptable disintegrating and wetting agents. The liquid preparations generally in the form of aqueous or oily solutions or suspensions, may contain conventional additives such as suspending or disperding agents.

For topical use the compounds of the present invention may also be prepared in suitable forms for absorption through the mucous membranes of the nose and throat or bronchial tissues and may conveniently take the form of liquid sprays or inhalants, lozenges, or throat paints. For medication of the eyes or ears, the preparation may be presented in liquid or semi-solid form. Topical applications may be formulated in hydrophobic or hydrophilic bases as ointments, creams, lotions, paints, or powders.

Compositions for injection may take such forms as suspensions, solutions, or emulsions in oily or aqueous vehicles, and may contain formulatory agents such as suspending, stabilizing and/or dispersing agents. Alternatively, the active ingredient may be in powder form for reconstitution, at the time of delivery, with a suitable vehicle, such as sterile water.

The amount of active principle to be administered depends on various factors such as the size and condition of the subject to be treated the route and frequency of administration, the particular compound employed and the causative agent involved. The compounds of the present invention are generally effective at a daily dosage comprised between about 5 and about 500 mg of active ingredient per Kg of body weight, preferably divided in 2 to 4 administrations per day.

Particularly desirable compositions are those prepared in the form of dosage units containing from about 30 to about 1500 mg per unit.

Besides their activity as medicaments, the compounds of the present invention can be used as bactericides in

industrial applications, e.g. preservatives of food, disinfectants and in other industrial systems where control of bacterial growth is desired.

The following examples further illustrate the present invention but in no way they have to be interpreted as a limitation to the scope thereof.

Preparation of the starting materials

A) Preparation of the bicyclic ketoesters of formula II.

Example 1

Trans- (±)-6-ethyl-3,7-dioxo-1-azabicyclo/3.2.0/heptan--2-carboxylic acid, (4-nitrophenyl)methyl ester (II : $R=CH_3-CH_2-$ ; $R_1=-CH_2-$ 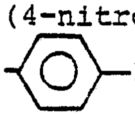 $-NO_2$)

a) Preparation of 1,3-hexadien-1-ol-acetate (Chart I - IV R=ethyl)

To a mixture of trans-2-hexenal (84.4 g; 0.86 mole) and acetic anhydride (102 g, 1 mole) 8 g (0.065 mole) of 4-dimethylaminopyridine and 105 g (1.04 mole) of triethylamine are added. The mixture is heated to 80°C for two hours and then, after cooling at room temperature , is added with 200 ml of chloroform. The organic layer is washed with two portions of 100 ml of ice-water and then with 30 ml of aqueous sodium bicarbonate. The chloroform layer is dried over $MgSO_4$. Evaporation of the solvent under vacuum yields a residue which is distilled at 80°C/60 mmHg. Yield 106 g N.M.R. and I.R. spectra confirm the assigned structure. The product is a mixture of four isomers containing more than 65% of the Δ-3,4-(E)-isomers.

b) Preparation of 4-/2-(acetyloxy)ethenyl/-3-ethyl-2-azetidinone (Chart I, VI R=ethyl)

1,3-Hexadien-1-ol acetate (105 g; 0.75 mole) is dissolved in 140 ml ethyl ether under argon atmosphere at -15°C. To this solution, chlorosulfonyl isocyanate (126 g; 0.984 mole) in ethyl ether (126 ml) is added under stirring.

Once the addition is complete, stirring at 0°C is maintained for 3 hours and then the solution is dripped into a mixture of 225 g (1.75 mole) of sodium sulfite and 375 g (2.15 mole) of potassium hydrogen phosphate in 1100 ml of water and 900 ml of ethyl ether. The mixture is stirred for 45 minutes on cooling with ice and adding solid bicarbonate to maintain the pH value at 6. The organic phase is separated and the aqueous layer is extracted with two 400-ml portions of ether. The combined organic layers are dried over magnesium sulfate and then evaporated to dryness under vacuum. The residue is suspended in 700 ml of petroleum ether and the mixture is stirred for 16 hours. After decantation, the organic solvent is removed and the residue is dried under reduced pressure yielding 46 g of a product which boils at 120°C/0.1 mmHg. Gas chromatographic assay shows that the product consists of a mixture of four isomers (trans -(Z), trans-(E), cis-(Z), cis-(E)). N.M.R. and I.R. spectra confirm the assigned structure. The single components may be separated through preparative HPLC. The ratio of the desired trans isomers pair in the mixture is of about 80%.

c) Preparation of 3-ethyl-4-/2-(hydroxy)ethyl/-2-azetidine (Chart I, II R=ethyl)

The crude 4-/2-(acetyloxy)ethenyl/-3-ethyl-2-azetidinone obtained according to step b) above (46 g) is dissolved in 600 ml of ethyl acetate and, after addition of 1.6 g of 20% palladium on carbon catalyst, is hydrogenated at room temperature with a pressure of 3 atmospheres of hydrogen gas in a Parr autoclave. After three hours,

the autoclave is discharged and the catalyst is filtered off. Evaporation of the solvent yields 46.5 g of 4-[2-(acetyloxy)ethyl]-3-ethyl-2-azetidinone which boils at 110$^{\circ}$C/0.03 mmHg. Gas chromatographic analysis of the product shows that the <u>trans</u> and <u>cis</u> isomers are in a 8:2 ratio. The isomers can be separated by preparative HPLC. The I.R. and N.M.R. data are in agreement with the assigned structure.
The crude 4-[2-(acetyloxy)ethyl]-3-ethyl-2-azetidinone is stirred with 9.7 g of potassium carbonate in 880 ml of absolute methanol at room temperature for 30 minutes. The mixture is neutralized with acetic acid and then evaporated to dryness at 50$^{\circ}$C under <u>vacuum</u>. The residue is suspended in a saturated aqueous solution of sodium chloride and the mixture is extracted with four 150-ml portions of ethyl acetate. The combined organic layers are dried over $MgSO_4$ and then evaporated to dryness yielding 34 g of 3-ethyl-4-[2-(hydroxy)ethyl]-2-azetidinone which contains 80% of the <u>trans</u> isomer (estimated by N.M.R.).
The I.R. and N.M.R. data confirm the assigned structure.

d) Preparation of <u>trans</u>-(±)-3-ethyl-4-oxo-2-azetidineacetic acid, I.
The crude 3-ethyl-4-[2-(hydroxy)ethyl]-2-azetidinone (34 g; 0.237 mole) obtained according to step c) is added to 63 g (0.394 mole) of potassium permanganate and 79 g (0.59 mole) of potassium dihydrogen phosphate in 870 ml of water. The mixture is maintained under stirring at 15$^{\circ}$C for 22 hours, then 220 ml of a saturated solution of sodium metabisulfite are added

thereto maintaining the value of the pH at about 3 by the gradual addition of 220 ml of 10% sulfuric acid. The mixture is stirred under cooling at 5°C until a clear solution is obtained (about 30 minutes), then, after saturation with ammonium sulfate, it is extracted with four 250-ml portions of ethyl acetate. The organic extracts are pooled together and dried over $MgSO_4$. Concentration of the ethyl acetate solution to 100 ml and cooling overnight in a refrigerator gives a crystalline precipitate which is recovered by filtration. Yield 16.43 g of trans-(±)-3-ethyl-4-oxo--2-azetidineacetic acid which melts at 99-101°C. Concentration of the mother liquors yields 2.23 g of the same product.

e) Preparation of trans-(±)-3-ethyl-4,β-dioxo-2-azetidinbutanoic acid, (4-nitrophenyl)methyl ester (XIII $R=CH_3CH_2-R_4= -CH_2$- 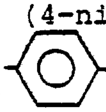 $-NO_2$)

A mixture of 3-ethyl-4-oxo-2-azetidineacetic acid (21 g), 1,1'-carbonyldiimidazole (24.32 g) and anhydrous tetrahydrofuran (360 ml) is stirred for 2/$\frac{1}{2}$ hours at room temperature, then mono-p-nitrobenzylmalonate magnesium salt (81.1 g) is added and stirring is continued for 19 hours at room temperature. The mixture is poured into 5% HCl (600 ml) and extracted with methylene chloride (four 450-ml portions).

The organic extracts are combined, washed with 10% $KHCO_3$ (two 200-ml portions), with brine (200 ml) and dried over $MgSO_4$. The solvent is removed under vacuum and the obtained residue is triturated with diethyl ether (60 ml), filtered and dried in vacuo at room temperature

yielding the desired keto-ester XIII ($R=CH_3CH_2$) (31.6 g) which melts at 88-90°C.

Preparation of mono p-nitrobenzylmalonate magnesium salt.

1st Step - The mono-para-nitrobenzylmalonate has been prepared by direct esterification of malonic acid with para-nitro-benzyl chloride according to the following procedures:

A solution of dry malonic acid (104 g, 1 mole) in dimethylformamide (290 ml) is treated at 15-20°C with triethylamine (139 ml, 1 mole). Under stirring, a solution of para-nitrobenzyl chloride (171.6 g, 1 mole) in dimethylformamide (400 ml) is added, followed by solid potassium iodide (25 g, 0.15 moles). After three days under stirring, the reaction mixture is diluted with methylene chloride (1 l) and slowly treated with 10% solution of potassium bicarbonate (2 l). The aqueous phase is acidified with 18% hydrochloric acid (420 ml) and the precipitate is filtered by suction, washed with water and dried to give 70 g (29.3%) of mono-para-nitrobenzylmalonate; M.p. 99-101°C.

2nd Step - A dry 3-l flask equipped with a mechanical stirred is charged with 145.85 g of mono-p-nitrobenzylmalonate (0.61 moles) and 1365 ml of anhydrous tetrahydrofuran. After 15 minutes 35.4 g of magnesium ethoxide (0.31 moles) are added and the reaction mixture is stirred for two hours. The resulting clear solution is then added in one hour to 2 liters of ethyl ether kept at $0^{\circ}C$ by an ice bath. After a further hour at $0^{\circ}C$ the resulting solid is filtered by suction and dried in vacuo to give 144 g (94%) of magnesium mono-p-nitrobenzylmalonate.

f) Preparation of trans-(±)-α-diazo-3-ethyl-4,β-dioxo-2--azetidinebutanoic acid, (4-nitrophenyl)methyl ester (XIV $R=CH_3CH_2$- $R_4=CH_2$- 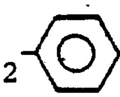 -$NO_2$)

To a mixture of the keto ester obtained in step e) above (30 g), p-carboxybenzenesulfonylazide (22.5 g) and acetonitrile (300 ml), cooled at $5^{\circ}C$, triethylamine (42 ml) is dropwise added over a period of fifteen minutes. When about one half of the triethylamine has been added, a clear solution results from which a white solid soon precipitates. The temperature is then allowed to rise at about $20^{\circ}C$ in half an hour. The white solid is filtered and whased with acetonitrile (50 ml). The combined solutions are evaporated in vacuo at room teperature and the residue is dissolved in methylene chloride (300 ml), washed with 8% $NaHCO_3$ (100 ml) and with water (100 ml). The organic phase is dried over $MgSO_4$ and concentrated in vacuo to a syrupy consistence. Diethyl ether (250 ml) is slowly added under magnetical stirring and stirring is continued for one hour at $0^{\circ}C$.

The product is collected by suction filtration and dried _in vacuo_ at room temperature yielding 29.16 g of the desired diazo-keto ester XIV ($R=CH_3CH_2-$) which melts at 115-18$^\circ$C.

g) Preparation of trans-(±)-6-ethyl-3,7-dioxo-1-aza-bicyclo/3.2.0/heptan-2-carboxylic acid, (4-nitrophenyl) methyl ester (XV $R=CH_3CH_2-$ $R_4= -CH_2-$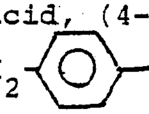$-NO_2$)

A solution of trans(±)-α-diazo-3-ethyl-4,β-dioxo-2-azetidinebutanoic acid, (4-nitrophenyl)methyl ester (30 g) prepared as described in step f) above in 1,2-dichloroethane (350 ml) is dripped into a solution of rhodium acetate (0.3 g) in 1,2-dichloroethane (50 ml) heated to the reflux temperature. The mixture is refluxed for additional 30 minutes while the progress of the reaction is followed by the nitrogen evolution and thin layer chromatography on silicagel plates eluting with ethyl acetate:hexane 6:4. The reaction mixture is then cooled at 20$^\circ$C, washed with water (two 100-ml portions), dried over $MgSO_4$ and evaporated to dryness under reduced pressure.

The residue is dissolved in methylene chloride (20 ml) and the product is precipitated by slow addition of diethyl ether (150 ml) under magnetic stirring and cooling.. After one hour, the solid is collected by suction and dried _in vacuo_ at room temperature yielding 25.7 g (91%) of trans(±)-6-ethyl-3,7-dioxo-1-azabicyclo/3.2.0/heptan-2-carboxylic acid, (4-p-nitrophenyl)methyl ester; M.p. 106-109$^\circ$C.

Example 2

a) Trans(+)-6-ethyl-3,7-dioxo-1-azabicyclo/3.2.0./-heptan-2-carboxylic acid (4-nitrophenyl)methyl ester. M.p. 79-80°C, $[\alpha]_D$ = + 231° (C = 1 in EtOH), $[\alpha]_D$ = + 224.1° (C = 1 in $CHCl_3$).

b) Trans(-)-6-ethyl-3,7-dioxo-1-azabicyclo/3.2.0/heptan--2-carboxylic acid (4-nitrophenyl)methyl ester. M.p. 79-81°C, $[\alpha]_D$ = -228.2° (C = 1 in $CHCl_3$).

The above compounds are separately obtained by following the procedures decribed in Example 1 above but resolving the trans-(±)-3-ethyl-4-oxo-2-azetidineacetic acid obtained in step d) into the single enantiomers by the method illustrated below and separately performing steps e), f), and g), on the individual corresponding enantiomers.

Resolution of trans-(±)-3-ethyl-4-oxo-2-azetidinacetic acid with dextro and laevo-oxyphene.

a) Salt of trans-(+)-3-ehtyl-4-oxo-2-azetidinacetic acid with laevo oxyphene.

Trans-(+)-3-ethyl-4-oxo-2-azetidinacetic acid (15.7 g, 0.1 mole) is added to a solution of l-oxyphene (28.35 g, 0.1 mole) in refluxing acetone (176 ml). The resulting solution is allowed to stand at room temperature for four hours, at about 3°C for 24 hours and then at about -25°C for 16 hours. The precipitate which forms is recovered by suction filtration, washed with ice-cooled acetone and dried under vacuum at 40°C for one hour to give 11.15 g of a salt characterized by an $[\alpha]_D = -4.5°$ (C = 1 in $CHCl_3$). This salt is crystallized twice from acetone (45 and 40 ml respectively) keeping the solutions at room temperature for one hour, then at about 3°C for four hours and finally at about -25°C for 16 hours. The salt of trans-(+)-3-ethyl-4-oxo-2-azetidinacetic acid with laevo-oxyphene thus obtained (7.04 g) has m.p. 124-24.5°C and $[\alpha]_D = + 4.3°$ (C = 1 in $CHCl_3$).

b) Trans-(+)-3-ethyl-4-oxo-2-azetidinacetic acid

To a stirred mixture of the above salt (7.04 g), water (18 ml) and diethyl ether (40 ml), 1N NaOH (15.8 ml) is gradually added to bring the pH to about 8.3. The aqueous phase is separated and extracted with diethyl ether (two 20-ml portions). The ethereal layers are washed

with water (two 10-ml portions), dried over $Na_2SO_4$ and concentrated to dryness yielding l-oxyphene (4.29 g, 94.2%).

The combined aqueous layers are saturated with $(NH_4)_2SO_4$ (40 g), stirred for a few minutes and acidified to pH 3.7 with 2N $H_2SO_4$ (about 8 ml). The mixture is extracted with ethyl acetate (180 ml followed by four 60-ml portions) and the combined extracts are dried over $MgSO_4$ concentrated to about 15 ml at room temperature, under vacuum, and kept at about -25°C for 18 hours. The solid is collected by filtration and dried under vacuum at room temperature to give 2.32 g of trans-(+)-3-ethyl-4-oxo-2-azetidinacetic acid. M.p. 113-15°C; $[\alpha]_D$ = + 16° (C = 1 in EtOH) - Enantiomeric purity: ~98%.

By carrying out the resolution essentially as described above but crystallizing the salt of trans-(+)-3-ethyl-4-oxo-2-azetidinacetic acid with l-oxyphene once more from acetone, pure trans-(+)-3-ethyl-4-oxo-2-azetidinacetic acid characterized by $[\alpha]_D$ = + 16.6° (C =1 in EtOH) is obtained.

c) Crude trans (-)-3-ethyl-4-oxo-2-azetidinacetic acid

The mother liquors deriving from the salification step described in a) above are evaporated to dryness and the crude laevo acid is recovered by following the method described in b) above. The ethyl acetate solution is distilled to dryness under vacuum at room temperature to give 10.45 g of crude laevo acid. $[\alpha]_D$ = -3.81° (C = 1 in EtOH).

d) Salt of trans-(-)-3-ethyl-4-oxo-2-azetidinacetic acid with dextro-oxyphene

The crude laevo acid obtained in c) above (10.45 g, 66.5 mmole) is added to a solution of dextro-oxyphene (11.82 g, 66.5 mmole) in hot acetone (117 ml). This solution is allowed to stand at room temperature for 4 hours and at about 4°C for 18 hours. The solid which precipitates is recovered by filtration and dried under vacuum at 40°C for one hour to give 8.85 g of a salt characterized by m.p. 120-21°C and $[\alpha]_D = + 2.44°$ (C = 1 in $CHCl_3$). This salt is crystallized twice from acetone (44 and 30 ml respectively) keeping the solutions at room temperature for 4 hours and at about 3°C for 18 hours. The salt of trans-(-)-3-ethyl-4-oxo-2-azetidin-acetic acid with d-oxyphene, thus obtained, (5.45 g) has m.p. 124-25°C and $[\alpha]_D = -4.35°$ (C = 1 in $CHCl_3$).

e) Trans-(-)-3-ethyl-4-oxo-2-azetidinacetic acid

The free acid is obtained from the corresponding d-oxyphene salt (5.45 g) by following the procedure described in b) above. Yield: 1.79 g of trans-(-)-3-ethyl-4-oxo-2-azetidinacetic acid characterized by a m.p. of 113-15°C and $[\alpha]_D = -16°$ (C = 1 in EtOH). Enantiomeric purity : ~98%.
Pure trans-(-)-3-ethyl-4-oxo-2-azetidinacetic acid recovered after a further crystallization of the d-oxyphene salt prepared in d) above from acetone, has $[\alpha]_D = -16.6°$ (C= 1 in EtOH).

Example 3

Cis(±)-6-ethyl-3,7-dioxo-1-azabicyclo/3.2.0/heptan--2-carboxylic acid, (4-nitrophenyl)methyl ester. M.p. 128°C.

The compound of the title is obtained by following substantially the same procedure as in Example 1 above but separating the mixture of trans and cis isomers of 4-/2-(acetyloxy)ethenyl/-3-ethyl-2-azetidinone obtained in step b) by the method described below and performating the following steps e) to g), on the couple of cis isomers.

a) Cis-(±)-4-/2-(acetyloxy)ethenyl/-3-ethyl-2-azetidinone.

The mixture of four isomers of 4-/2-(acetyloxy)ethenyl/--3-ethyl-2-azetidinone (40 g, 2.18 x $10^{-1}$ mole) obtained in Example 1 b), is taken up with peroxide-free isopropyl ether (40 ml) and the mixture is stirred at room temperature for about 4 hours and allowed to stand at about 3°C for 8 hours. The solid is recovered by filtration and dried under vacuum to yield 4.4 g (11%) of the cis isomers pair. M.p. 102°C.

The N.M.R. and I.R. spectra confirm the assigned structure.

B) Preparation of the cysteamine derivatives

Example 4

2-(2-Thiazolylamino)-ethanethiol.

a) Acylating step

Thionyl chloride (79.9 g, 0.67 moles) was added dropwise at room temperature to 2-acetylmercaptoacetic acid (82 g, 0.61 moles), followed by a catalytic amount of dimethylformamide (0.5 ml). After the addition was completed, the reaction mixture was stirred at 50°C for 30' and at 100°C for 10 minutes. The reflux condenser was replaced with a Liebig condenser and the product was distilled at 90°/20 mm. The yield was 70.5 g (75.6%). 2-Aminothiazole (10 g, 0.1 moles) was dissolved in pyridine (50 ml) and dry methylene chloride (100 ml) in a 500-ml four necked flask placed on a magnetic stirrer.

A solution of acetylmercaptoacetyl chloride (15.2 g, 0.1 moles) in methylene chloride (50 ml) was slowly added to the stirred solution, keeping the temperature at 0-5°. After the addition was complete, the reaction mixture was stirred on an ice bath for one hour, then was poured in 600 ml of cold water. The solid product which separates was filtered *in vacuo*, washed with methylene chloride and dried to give 14.7 g of 2-(acetylmercaptoacetyl)-amino thiazole.

M.p. 177-178°C.

The combined organic phases were washed with dil. $NaHCO_3$ $H_2O$ and dried ($MgSO_4$) The solvent was distilled *in vacuo* and the residue was triturated with ethyl ether to give additional 2.9 g of the desired product.

M.p. 177-178°C. The total yield was 17.65 g (81.7%).

The starting 2-acetylmercaptoacetic acid was prepared adding acetyl chloride (187.5 g, 2.39 mole) dropwise in 30' to 2-mercaptoacetic acid (200 g, 2.17 mole), kept under stirring at room temperature. A vigorous evolution of hydrochloric acid took place. After 30' the reaction mixture was stirred at reflux for an additional hour.
The cooled mixture was distilled under reduced pressure through a short (8 cm) Vigreux column to give 170.38 g (58;5%) of 2-acetylmercaptoacetic acid, b.p. 150°/20 mm.

b) <u>Reductive step</u>
In a 500 ml, four-necked flask, purged with dry Argon, are placed 30 ml of dry THF and lithium aluminium hydride (2.64 g, 0.07 moles). The slurry was stirred at -5° while a warm solution of 2-(acetylmercaptoacetyl)-aminothiazole (5 g, 0.023 moles) in dry tetrahydrofuran (170 ml) was added dropwise over an one hour period.
The mixture was stirred 30' at 0° and 2 hours at room temperature, then was cooled in an ice both and decomposed by gradual addition of $H_2O$ (40 ml) followed by 8% HCl (150 ml). This mixture was extracted with diethyl ether (75 ml) and the aqueous phase was neutralized with $NaHCO_3$ sol. (300 ml).
The inorganic salts were filtered, washed with methylene chloride and the filtrate was extracted with methylene chloride (3 x 200 ml).
The combined organic phases were dried ($MgSO_4$) and concentrated to give 3.4 g of thiol, m.p. 66-68°.

A sample was distilled in a Kugelrohr apparatus: b.p. 110$^{\circ}$C/0.1 mmHg.

Example 5

2-(3-Methyl-2-pyridyl)amino-ethanethiol.

a) Acylating step

To a stirred solution of 2-amino-3-methylpyridine (32.5 g, 0.3 moles) in $CH_2Cl_2$ (150 ml), acetylmercaptoacetic acid (37.2 g, 0.28 moles) in $CH_2Cl_2$ (200 ml) was added, followed by dicyclobexylcarbodiimide, (61.3 g, 0.297 moles). The reaction mixture was stirred at room temperature overnight, dicyclohexylurea was filtered in vacuo and washed with $CH_2Cl_2$ (2 x 50 ml). The organic solution was washed with dil. $NaHCO_3$ (2 x 50 ml) and the basic products were extracted with 10% $H_2SO_4$ (4 x 70 ml). The aqueous phases were adjusted at pH 4.8 with 30% NaOH and extracted with $CH_2Cl_2$ (100 ml plus 3 x 60 ml). The combined organic phases were washed with $H_2O$ (2 x 50 ml) and dried ($MgSO_4$). The solvent was distilled in vacuo and the residue (48.15 g) was crystallized from $CH_2Cl_2$/diethyl ether to give 34.89 g (55.6%) of pure product, m.p. 76-78$^{\circ}$C.

b) Reductive step

The reductive step was carried out exactly as described in Example 4b) (66% yields). The end 2-(3-methyl-2-pyridyl)-aminoethanethiol, distilled in a Kugelrohor has b.p. 120$^{\circ}$C/0.05 mmHg.

Examples 6a) to 10a)

The compounds listed in Table II have been obtained by following the method described in Example 4a) or 5a) as indicated.

TABLE II

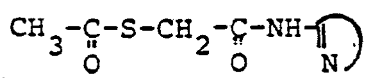

| Example No. | 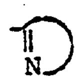 | Method of Example | M.p. or B.p. | % Yields |
|---|---|---|---|---|
| 6a) | 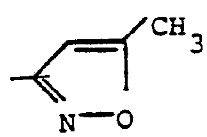 | 4a) | 182-184°C | 75 |
| 7a) | 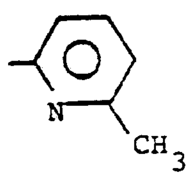 | 4a) | 140°C/0.1mmHg | 36 |
| 8a) | 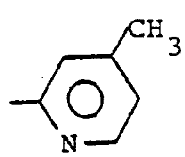 | 5a) | 93°C | 48 |

ctd.

TABLE II ctd.

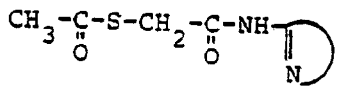

| Example No. | 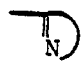 | Method of example | M.p. | %Yield |
|---|---|---|---|---|
| 9a) | 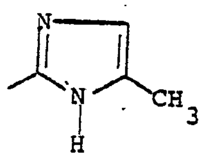 | 5 a) | 192°C | 32 |
| 10a) | 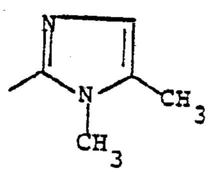 | 5 a) | 132-4°C | 46 |

Examples 6b) to 10b)

The compounds listed in Table III below have been obtained by following the procedure in Example 4b) but starting from the corresponding intermediate of Table II.

TABLE III

HS-$CH_2$-$CH_2$-NH-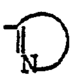

| Example No. | 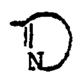 | M.p. or B.p. | % Yields |
|---|---|---|---|
| 6b) | 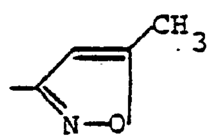 | 54-56°C | 98 |
| 7b) | 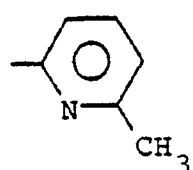 | 80°C/0.05 mmHg | 67 |
| 8b) | 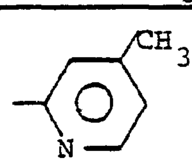 | 52-4°C | 97 |

a) Distilled in a Kugilrohor apparatus.

ctd.

TABLE III ctd.

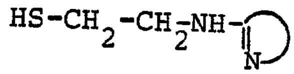

| Example No. | 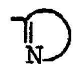 | M.p. or B.p. | % Yield |
|---|---|---|---|
| 9 b) | 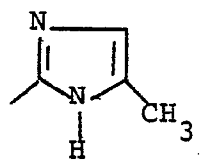 | - b) | - |
| 10 b) | 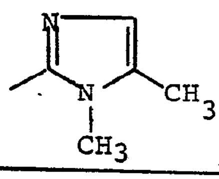 | - b) | - |

a) Distilled in a Kugelrohor apparatus

b) These intermediates, being unstable, were used at once in the next step described in Examples 11 and 12.

Due to the enhanced nucleophylicity of the 2-amino-imidazole moiety, the compounds of examples 9b) and 10b) could not be used directly in the reaction scheme of Chart I but a further protection was necessary. In particular as reported in Chart VI below, the substituted β-imidazolyl-cysteamines of examples 9b and 10b) were treated with p-nitrobenzylchloroformiate in acetonitrile and in the presence of di-isopropylethylamine and dimethylaminopyridine to give the bis-acylated derivatives. Selective removal of the mercapto protecting group with ammonia in methanol, afforded the protected β-imidazolyl cysteamine which were used in the condensation with the enol-phosphate III

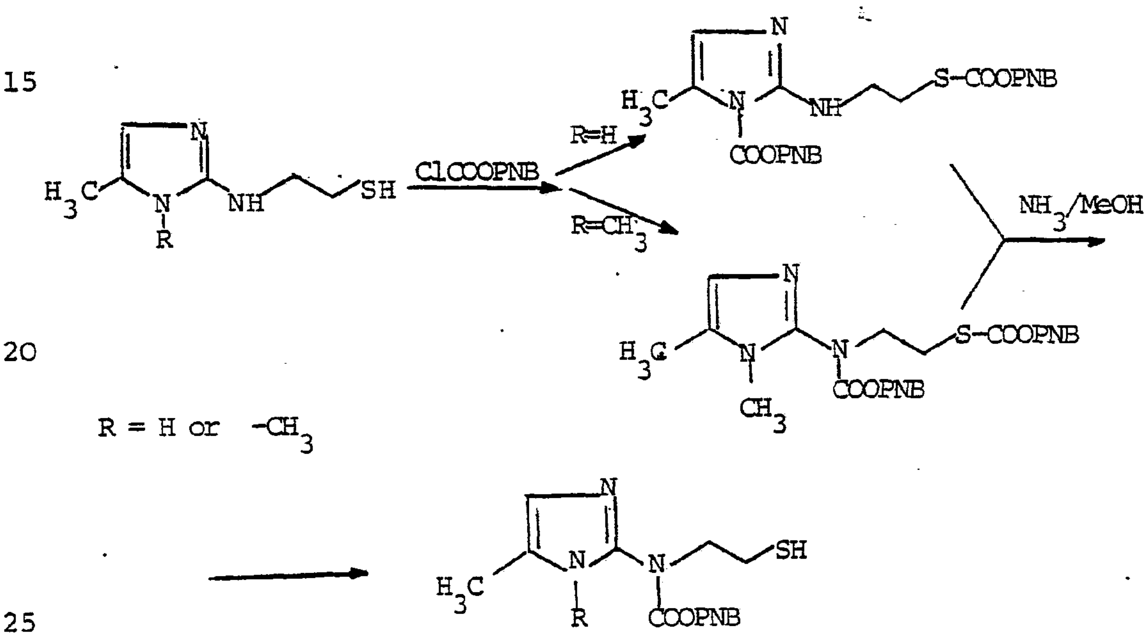

Example 11

2-/3,4-dimethyl-2-imidazolyl-(N-p-nitrobenzyloxycarbonyl) amino/-ethanethiol.

A solution of freshly prepared 2-/3,4-dimethyl-2-imidazolylamino/ethanethiol (example 10b)) (0.83 g, 4.84 mmoles), diisopropylethylamine (2.07 ml, 12.09 mmoles) and 4-dimethylaminopyridine (0.146 g, 1.209 mmoles) in dry acetonitrile (15 ml) is poured into a four-neck 100-ml flask, purged with dry Argon. A solution of p-nitrobenzylcarbonate (2.08 g, 9,68 mmoles) in dry acetonitrile (8 ml) is then added thereto at 0°C over a period of five minutes.

After 30 minutes at 0°C and 3 hours at room temperature, the solution is diluted with methylene chloride (60 ml) and washed with 3% HCl (30 ml), water (20 ml) and 8% sodium bicarbonate solution (20 ml). After drying over $MgSO_4$, the solvent is removed in vacuo and the residue is purified by chromatography on silica gel (30 g) eluting with methylene chloride containing from 0 to 20% of ethyl acetate. The oily compound thus obtained is dissolved in methanol (60 ml) containing 6% of ammonia and the obtained solution is stirred at 40-50°C for three hours under Argon.

The solvent is then evaporated off in vacuo leaving the compound of the title as an oily residue.

Example 12

2-/4-methyl-2-imidazolyl-(N-p-nitrobenzyloxycarbonyl)-amino/ethanethiol.

This compound is obtained by following substantially the same procedure of Example 11 but starting from the 2-/4-methyl-2-imidazolylamino/-ethanethiol of Example 9 b).

Preparation of the compounds of the invention

Example 13

Trans-(±)-6-ethyl-7-oxo-2-/[2-/(2-thiazolyl)amino/-ethyl/thio/-1-azabicyclo/3.2.0./hept-2-en-2-carboxylic acid, (4-nitrophenyl)methyl ester.

A solution of trans-(±)-6-ethyl-3,7-dioxo-1-azabicyclo-/3.2.0./heptan-2-carboxylic acid, (4-nitrophenyl)methyl ester (0.58 g, 1.74 mmoles) in dry acetonitrile (3 ml) was cooled at 0°C and treated with diisopropylethylamine (0.495 g, 3.83 mmoles) followed, after 5', by diphenylchlorophosphate (0.514 g, 1.91 mmoles). The mixture was stirred at 0°C for 20' (TLC: Silicagel, $CH_2Cl_2$: AcOEt 95/5), then a solution of 2-(2-thiazolylamino) ethanthiol (0.29 g, 1.82 mmoles) in acetonitrile (2 ml) was added dropwise over 10'. After 1 hour at 0°C, the solid product which separates was filtered *in vacuo*, washed with diethyl ether and dried to give 0.5 g (60.9%) of the thiazolyl derivative, (4-nitrophenyl)methyl ester m.p. 120-130°C.

1H-NMR($CDCl_3$): δ 1.07 (t, 3H, $J_{CH_3-CH_2}$ = 7 Hz, $CH_3$), 1.87 (m, 2H, $\underline{CH_2}-CH_3$), 3.0-3.4 (m, 5H, $CH_2$(4), $CH_2$(12), CH(6)), 3.63 (t, 2H, $J_{CH_2(11)-CH_2(12)}$=6.5 Hz, $CH_2$(11)), 3.95 (dt, 1H, $J_{H(5)-H(6)}$= 2.5 Hz, $J_{H(5)-CH_2(4)}$=9Hz, CH(5)), 5.25-5.52 (2d, NH, $J_{gem}$= 14 Hz, $CH_2O$), 5.58 (b, 1H, NH), 6.54 (d, 1H, $J_{H_4'-H_5'}$=3.5, H5'), 7.13 (d, 1H, $H_4'$), 7.69, (d, 2H J ortho=9 Hz C-Ar), 8.24 (d, 2H, N-Ar).

Examples 14 to 21

The compounds of Examples 14 to 21 have been prepared by following substantially the same procedure described in Example 13 but using the proper cysteamine derivatives. (The cysteamine derivatives used for preparing the compounds of Examples 14 and 15 are known products).

T A B L E IV

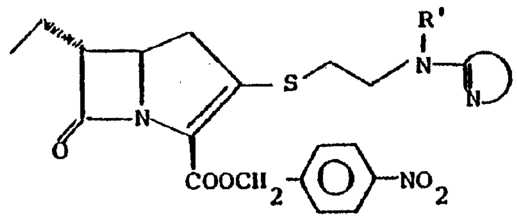

| Example No. | R' | 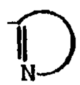 | m. p. °C | % Yields | U.V. ($CH_3OH$) $\lambda_{max}$ | $E^{1\%}_{1cm}$ | I.R. ($CDCl_3$) $cm^{-1}$ $\nu_{NH}$ | $\nu_{C=O}$ lactam | $\nu_{C=O}$ ester | $\nu_{C=C}$ $\nu_{C=N}$ | $\nu_{NO_2}$ |
|---|---|---|---|---|---|---|---|---|---|---|---|
| 14 | H | 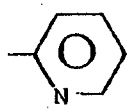 | 113-117[a)] | 88 | 244<br>268<br>315 | 427.5<br>252.5<br>322.5 | 3460 | 1774 | 1700 | 1600<br>1571<br>1550 | 1523<br>1350 |
| 15 | H | 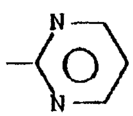 | 84-85[a)] | 65 | 227<br>259<br>317 | 444<br>247<br>277 | 3455 | 1773 | 1700 | 1605(s)<br>1586<br>1567 | 1520<br><br>1350 |

continued.....

continued..

**T A B L E IV**

| Example No. | R' | 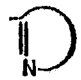 | m. p. °C | % Yield | U.V. ($CH_3OH$) $\lambda_{max}$ | U.V. ($CH_3OH$) $E^{1\%}_{1cm}$ | $\nu_{NH}$ | I.R. ($CDCl_3$) $\nu_{C=O}$ lactam | I.R. ($CDCl_3$) $\nu_{C=O}$ ester | $cm^{-1}$ $\nu_{C=C}$ $\nu_{C=N}$ | $cm^{-1}$ $\nu_{NO_2}$ |
|---|---|---|---|---|---|---|---|---|---|---|---|
| 16 | H | 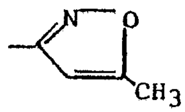 | 133[a)] | 65 | 264<br>318 | 245<br>271 | 3460 | 1772 | 1700 | 1626<br>1608<br>1535(s) | 1521<br><br>1348 |
| 17 | H | 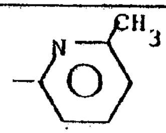 | 132[b)] | 47 | 246<br>313 | 400<br>337 | 3460 | 1775 | 1700 | 1601<br>1550<br>1500 | 1521<br>1350 |
| 18 | H | 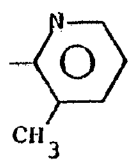 | 105-113[a)] | 90 | 240<br>267<br>309 | 409<br>275<br>321 | 3466 | 1772 | 1700 | 1600<br>1585<br>1550<br>1500 | 1522<br>1350 |
| 19 | H | 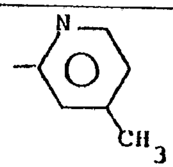 | 135-145[a)] | 92 | 247<br>268<br>315 | 415<br>n.d.<br>288 | 3465 | 1775 | 1700 | 1615<br>1550<br>1490 | 1522<br>1350 |

continued... <u>T A B L E IV</u>

| Example No. | R' | 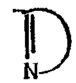 | M.p. °C | Yields % | U.V. ($CH_3OH$) λ max | U.V. ($CH_3OH$) $E^{1\%}_{1cm}$ | I.R. ($CDCl_3$) $cm^{-1}$ $\nu_{NH}$ | $\nu_{C=O}$ lactam | $\nu_{C=O}$ ester | $\nu_{C=C}$ $\nu_{C=N}$ | $\nu_{NO_2}$ |
|---|---|---|---|---|---|---|---|---|---|---|---|
| 20 | -H | 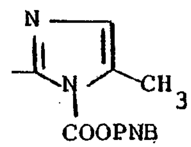 | 134-5 a) | 17 | 265<br>316 | 409<br>189 | | 1780 | 1720 | 1610<br>1580<br>1560 | 1530<br>1350 |
| 21 | 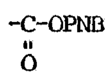 | 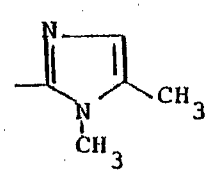 | 150-2 c) | 12 | 268<br>318 | 314<br>184 | 3420 | 1780 | 1738 | 1610<br>1588 | 1530<br>1350 |

a) Crystallized from methylene chloride/diethyl ether

b) Crystallized from ethanol

c) Crystallized from acetonitrile

0093915

Example 22

Trans-(±)-6-ethyl-7-oxo-2-/[2-(2-pyridylamino)ethyl]-thio/-1-azabicyclo/3.2.0/hept-2-en-2-carboxylic acid S-oxide, (4-nitrophenyl)methyl ester.

A solution of the compound of Example 14 (0.45 g, 0.96 mmoles) in dry $CH_2Cl_2$(25 ml) was chilled to -30° and m-chloroperbenzoic acid (0.182 g,1.06 mmoles) dissolved in $CH_2Cl_2$ (15 ml) was added with stirring in 30'. The reaction was monitored by t.l.c. (toluene/acetone 1:1) and after 1 hour at -30°, when oxidation was completed the solution was washed twice with 1/15 M phosphate buffer pH 8 and dried (Mg $SO_4$). Evaporation of the solvent afforded 0.45 g of crude, rather unstable sulfoxide, m.p. 135-140° dec.

1H-NMR ($CDCl_3$) : δ 1.04-105 (2t, 3H, $J_{CH_3-CH_2}$ = 7Hz, $CH_3$), 1.85 (m, 2H, $CH_2$-$CH_3$) 3.0-3.7 (m, 5H, $CH_2$(4), $CH_2$(12), CH(6)), 3.8-3.9 (m, 2H, $CH_2$(11)), 4.03-4.11 (2dt, 1H, $J_{H_5-H_6}$=2.5 Hz, $J_{H_5-CH_2(4)}$ = 9Hz, $H_5$), 5.00-5.05 (2t, 1H, $J_{NH-CH_2(12)}$=5.5Hz, NH), 5.1-5.5 (m, 2H, $CH_2O$), 6.43-6.45 (2d, 1H, $J_{H_{3'}-H_{4'}}$ = 8, $H_{3'}$), 6.63 (dd, 1H, $J_{ortho}$ = 8-5, $H_{5'}$), 7.43 (dd, 1H, $H_{4'}$), 7.62-7.64 (2d, 2H, $J_{ortho}$ =8Hz, C-Ar) 8.12 (d, 1H, $H_{6'}$), 8.25-8.26 (2d, 2H, N-Ar).

U.V. absorption maxima ($CH_3OH$): 242 nm ($E^{1\%}_{1cm}$ = 420), 267 nm ($E^{1\%}_{1cm}$ 259) and 290 nm ($E^{1\%}_{1cm}$ = 243)

I.R. main peaks in $CDCl_3$($cm^{-1}$) : 3460 ($\nu$NH),1787 ($\nu$C=O β-lactam), 1720 ($\nu$C=O ester), 1605, 1585, and 1500 ($\nu$C=O and $\nu$C=N), 1525 and 1350 ($\nu NO_2$), 1050 ($\nu$S→O).

Example 23

Trans-(±)-6-ethyl-7-oxo-2-[[2-(2-(2-pyrimidinyl)ethyl]-thio]-1-azabicyclo[3.2.0]hept-2-en-2-carboxylic acid S-oxide, (4-nitrophenyl)methyl ester.

The compound of the title is obtained as an oily product by following the procedure described in the foregoing example but starting from the compound of Example 15.

Example 24

Trans-(±)-6-ethyl-7-oxo-2-[[2-[(2-thiazolyl)amino]-ethyl]thio]-1-azabicyclo[3.2.0]hept-2-en-2-carboxylic acid potassium salt.

A solution of the (4-nitrophenyl)methyl ester of Example 13 (150 mg, 0.316 mmoles) in tetrahydrofuran (15 ml) and potassium phosphate buffer 1/15 M pH 8.5 (15 ml, 1 mmoles of $PO_4^{3-}$) is hydrogenated in the presence of 10% Pd on carbon (150 mg) at room pressure and temperature for 2 hours. The catalyst is filtered on celite (1 g) and washed with water (10 ml). The organic solvent is removed at 25°C/1 mm and the resulting solution is extracted with $CH_2Cl_2$ (10 ml), re-filtered on celite (1 g) and black carbon (0.3 g), washing the cake with water (10 ml).

The combined aqueous solution (30 ml, $\lambda_{max}$=300μm, ~ 3 mg/ml) is kept in the refrigerator overnight, then it is charged on a column of QAE-Sephadex A-25 (30 ml) and developed with a linear gradient between solution A and B (total eluent 300 ml).

Solution A: Potassium buffer 0.01 M, pH 7.9 (150 ml)
Solution B: 1.5% K Cl in Potassium phosphate buffer 5 mM, pH 7.9 (150 ml).

Each 150 drops fraction is monitored by HPLC and those containing the product (13 ÷ 18; pH 7.7 ) are combined and treated with Amberlite ® XAD-2 (10 g). This suspension is charged on a column of Amberlite® XAD-2 (10 g in 40 ml of potassium phosphate buffer 5 mM, pH 7.9 containing 0.5% of KCl) and eluted with : 1) distilled water ( 50 ml), 2) methanol/water 1:1 (100 ml), 3) methanol/water 7:3 (100 ml). The combined fractions are concentrated at 28°C/1 mm up to 10 ml, filtered on Millipore ® and lyophilized to afford 54.3 mg (45%) of the product, as a white solid.

Examples 25 to 34

The compounds listed in Table V are prepared by following essentially the same procedure as in Example 22 but starting from the appropriate (4-nitrophenyl)methyl esters.

# TABLE V

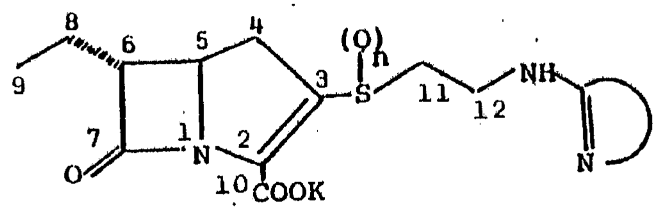

| Example No | 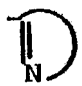 | n | Yield % | U.V. max | U.V. $E^{1\%}_{1cm}$ | HPLC a) Eluent b) | HPLC a) $R_t$ (min) | I.R. (KBr) $cm^{-1}$ |
|---|---|---|---|---|---|---|---|---|
| 25 | 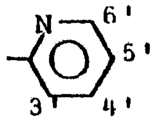 | O | 79 | 237<br>303 | 226.5<br>196.3 | 6/4 | 3.00 | 1760, 1600 |
| 26 | 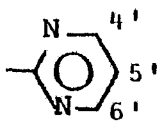 | O | 69 | 302 | 185 | 6/4 | 2.40 | 1755, 1585,<br>1530 |
| 27 | 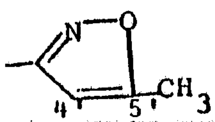 | O | 38 | 300 | 276 | 7/3 | 5.11 c) | 1755,1630,<br>1600,1555 |
| 28 | 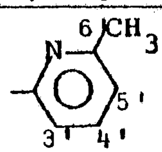 | O | 30 | 237<br>303 | 352<br>379 | 6/4 | 7.00 | 1755,1600<br>1515 |

continued... **T A B L E V**

| Example No. | 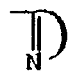 | n | Yield % | U.V. | | HPLC a) | | I.R. (KBr) $cm^{-1}$ |
|---|---|---|---|---|---|---|---|---|
| | | | | $\lambda_{max}$ | $E^{1\%}_{1cm}$ | Eluent b) | $R_t$ (min) | |
| 29 | 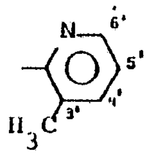 | 0 | 11 | 238<br>300 | 312<br>330 | 6/4 | 5.88 c) | n.d. |
| 30 | 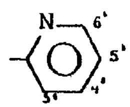 | 1 | 10 | 298 | 168 | 6/4 | 1.88 c) | n.d. |
| 31 | 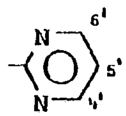 | 1 | 8 | 292 | 113 | 6/4 | 1.55 c) | n.d. |
| 32 | 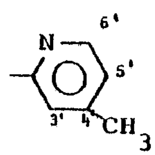 | 0 | 25 | 240<br>303 | 310<br>330 | 6/4 | 6.35 c) | n.d. |

continued...

**T A B L E V**

| Example No. | 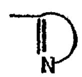 | n | Yield % | U.V. | | HPLC a) | | I.R. (KBr) $cm^{-1}$ |
|---|---|---|---|---|---|---|---|---|
| | | | | $\lambda$ max | $E^{1\%}_{1cm}$ | Eluent b) | $R_t$ (min) | |
| 33 | 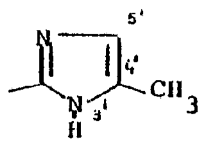 | 0 | 4 | n.d. | - | 65/35 | 3.38 c) | n.d. |
| 34 | 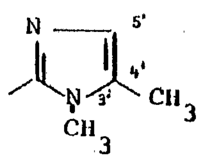 | 0 | 28 | 298 | 210 | 65/35 | 3.60 c) | n.d. |

n.d. = Not determined

a) Reverse phase HPLC analyses were performed using an Altex 110A pump equipped with a 4mm i.d.x. 25cm Licrosorb RP18 to μm column and a PE LC15 (254 μm) detector at a flow rate of 2/ml min.

b) Ratio between phosphate buffer and methanol at pH 7.5-7.8.

c) Determination using a H.P. 3390A Integrator.

The compounds of Examples 24 to 34 show the following main peaks in their $^{1}H$-NMR Spectra:

| Comp. of Example No. | $CH_3(9)$,t $J_{CH_3(9)-CH_2(8)}$ =7Hz | $CH_2(8)$,m | $CH_2(4)+CH(6)$ $+CH_2(12)$,m | $CH_2(11)$,t $JCH_2(11)-CH_2(12)$= 6 Hz | CH(5), d t JCH(5)-CH(6)=2.5 Hz JCH(5)-$CH_2(4)$=9Hz | 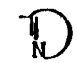 |
|---|---|---|---|---|---|---|
| 24 | 1.01 | 1.82 | 2.9-3.3 | 3.67 | 4.01 | 6.84(d, 1H,$J_{H_{4'}-H_{5'}}$=3.5,$H_{4'}$ 7.26 (d, 1H, $H_{5'}$) |
| 25 | 0.98 | 1.75 | 2.8-3.2 | 3.58 | 3.84 | 6.73(m, 2H, $H_{3'}$+$H_{5'}$);7.62 (dd, 1H,$J_{orto}$=5.5,$H_4$); 7.95 (d,1H,$J_{ortho}$= 5.5,$H_{6'}$). |
| 26 | 0.99 | 1.78 | 2.9-3.2 | 3.63 | 3.90 | 6.73(dd, 1H,$J_{ortho}$=6, $H_5$); 8.30 (d, 2H, $H_{4'}$+$H_{6'}$) |
| 27 | 1.01 | 1.80 | 2.9-3.2 | 3.40 | 3.97 | 2.30 (s,3H. $CH_3$ in 5'); 5.87 (s, 1H, $H_4$') |
| 28 | 0.99 | 1.78 | 2.8-3.2 | 3.62 | 3.89 | 2.29 (s, 3H, $CH_3$ in 6'); 6.57 (d,2H,$J_{ortho}$=6.5, $H_{3'}$ +$H_{5'}$); 7.61)(t, 1H,$H_4$') |

Continued....

| | | | | | | |
|---|---|---|---|---|---|---|
| 29 | 0.93 | 1.70 | 2.54-3.2 | 3.65 | 3.62 | 2.08(s,3H,$CH_3$ in 3');6.77 (dd, 1H, $J_{ortho}$=5-7, $H_{5'}$), 7.48 (d, 1H, $H_{4'}$) 7.93 (d, 1H, $H_{6'}$). |
| 30 | 0.97-0.98 | 1.78 | 2.9-3.5 | 3.77 ($J_{CH_2(11)-CH_2(12)}$=6.5) | 4.02-4.12 ( 2 d t) | 6.6-6.9(m, 2H, $H_{3'}$ and $H_{5'}$), 7.64 (dd, 1H, $J_{orto}$=8Hz, $H_{4'}$) 8.03(d, 1H, $J_{orto}$=6Hz, $H_{6'}$) |
| 31 | 0.99 | 1.79 | 3.0-3.7 | 3.83 ($J_{CH_2(11)-CH_2(12)}$=6.5 | 4.05-4.20 ( 2 d t) | 6.7-6.8 (m, 1H, $H_{5'}$), 8.3-8.4 (m, 2H, $H_{4'}$ and $H_{6'}$) |

Continued......

| | | | | | | |
|---|---|---|---|---|---|---|
| 32 | 0.98 | 1.76 | 2.8-3.2 | 3.62 | 3.84 | 2.31(s, 3H, $CH_3$ in 4'); 6.68(s, 1H, $H_3$,); 6.69 (d, 1H, $J_{ortho}$=5.5 Hz, $H_5$'); 7.82 (d, 1H, $H_6$') |
| 33 | 1.00 | 1.80 | 2.9-3.3 | 3.56 | 3.95 | 2.12(s, 3H, $CH_3$-Im.), 6.48 (s,1H, $H_5$') |
| 34 | 1.00 | 1.79 | 2.8-3.2 | 3.58 | 3.91 | 2.14(s, 3H, $CH_3$-in 4'); 3.33 (s, 3H, $CH_3$ in 3'); 6.55 (s, 1H, $H_5$') |

Recorded at 270 MHz with a Brücker spectrometer in $D_2O$ with TSP as an internal standard
ppm = $\delta$

## Preparation of pharmaceutical compositions

### Example 35

A gelatine capsule is filled with a blend of
250 mg of the compound of Example 25,
25 mg of talc,
25 mg of sodium carboxymethylcellulose and
90 mg of starch

### Example 36

A gelatin capsule is filled with a blend of
400 mg of the compound of Example 24,
50 mg of starch,
20 mg of talc, and
10 mg of magnesium stearate

### Example 37

A parenteral solution is prepared with 500 mg of the compound of Example 25 dissolved in 2 ml of Sterile Water for Injection

### Example 38

A topical ointment is prepared with
200 mg of the compound of Example 26
600 mg of polyethylene glycol 4000 U.S.P.
1.2 g of polyethylene glycol 400 U.S.P.

Following the procedure described in the foregoing text and Examples, the following compounds listed in Table VI are obtained.

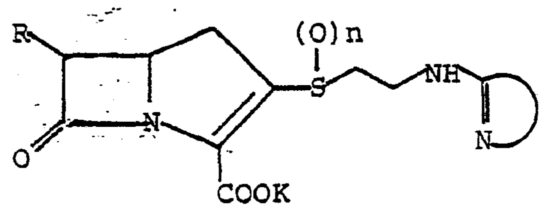

TABLE VI

| Compound No. | R | n | 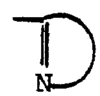 |
|---|---|---|---|
| 1 | $CH_3CH_2-$ | 0 | 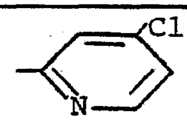 |
| 2 | $CH_3CH_2-$ | 0 | 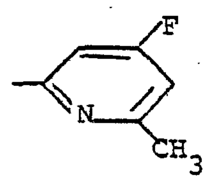 |
| 3 | $CH_3CH_2-$ | 0 | 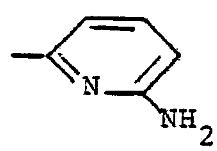 |
| 4 | $CH_3$<br>\|<br>$CH_3-CH-$ | 0 | 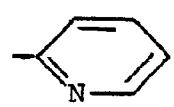 |
| 5 | $CH_3$<br>\|<br>$CH_3-CH-$ | 0 | 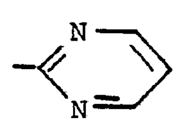 |

Continued...

| Compound No. | R | n | 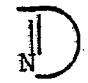 |
| --- | --- | --- | --- |
| 6 | $CH_2$=CH- | 0 | 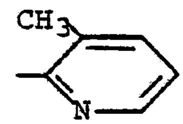 |
| 7 | $CH_3CH_2$- | 0 | 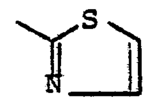 |
| 8 | $CH_3$-$CH_2$- | 0 | 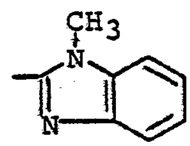 |
| 9 | $CH_3$-$CH_2$- | 0 | 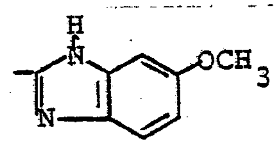 |
| 10 | $CH_3$-$CH_2$- | 0 | 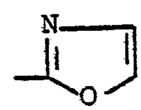 |
| 11 | $CH_3$-$CH_2$- | 1 | 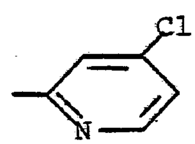 |
| 12 | $CH_3$-$CH_2$- | 0 | 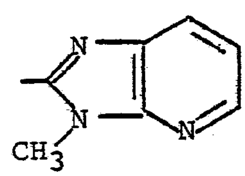 |
| 13 | $CH_3$-$CH_2$- | 0 | 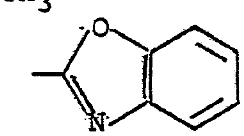 |
| 14 | $CH_3$-$CH_2$- | 0 | 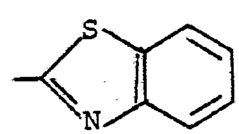 |

Continued.....

| Compound No. | R | n | 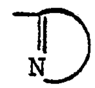 |
| --- | --- | --- | --- |
| 15 | $CH_3-CH_2-$ | 0 | 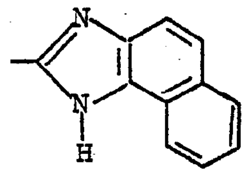 |
| 16 | $CH_3-CH_2$ | 1 | 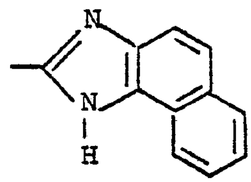 |
| 17 | $CH_3-\overset{\overset{CH_3}{\mid}}{CH}-$ | 0 | 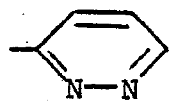 |
| 18 | $CH_3-\overset{\overset{CH_3}{\mid}}{CH}-$ | 0 | 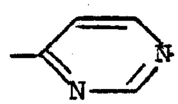 |
| 19 | $CH_3-CH_2-$ | 0 | 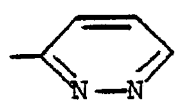 |
| 20 | $CH_3-CH_2-$ | 0 | 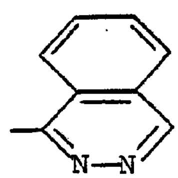 |
| 21 | $CH_3-CH_2-$ | 0 | 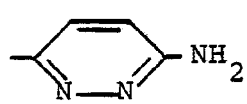 |
| 22 | $CH_3-CH_2-$ | 0 | 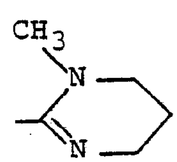 |

Continued.....

| Compound No. | R | n | 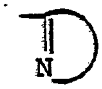 |
|---|---|---|---|
| 23 | $CH_3-CH_2-$ | O | 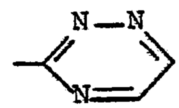 |
| 24 | $CH_3-CH_2-$ | O | 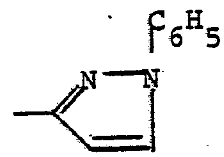 |
| 25 | $CH_3-CH_2$ | O | 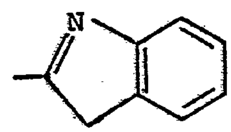 |
| 26 | $CH_3-CH(CH_3)-$ | O | 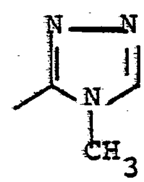 |
| 27 | $CH_3-CH_2-$ | O | 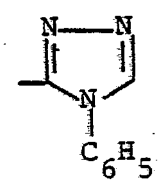 |
| 28 | $CH_3-CH_2-$ | O | 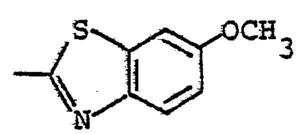 |
| 29 | $CH_3-CH(CH_3)-$ | O | 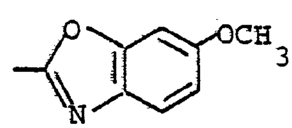 |
| 30 | $CH_3-CH_2-$ | O | 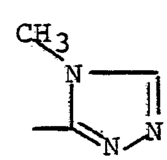 |

Continued.....

| Compound No. | R | n | 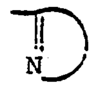 |
|---|---|---|---|
| 31 | $CH_3CH_2$- | O | 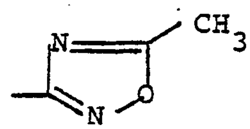 |
| 32 | $CH_3$-$CH_2$- | O | 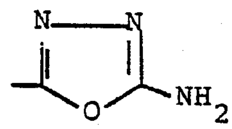 |
| 33 | $CH_3$-$CH_2$- | O | 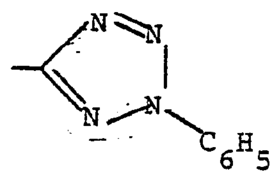 |
| 34 | $CH_3$-$CH_2$- | O | 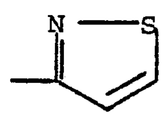 |
| 35 | $CH_3$-$CH_2$- | O | 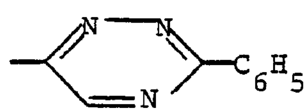 |
| 36 | $CH_3$-$CH_2$- | O | 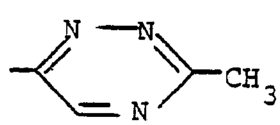 |
| 37 | $CH_3$-$CH_2$- | O | 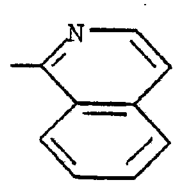 |

Continued.....

| Compound No. | R | n | 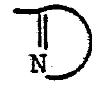 |
|---|---|---|---|
| 38 | $CH_3-CH_2-$ | O | 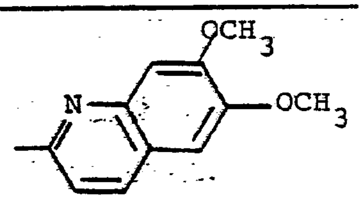 |
| 39 | $CH_3-CH_2-$ | O | 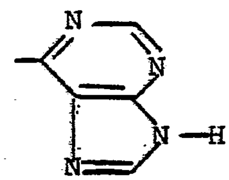 |
| 40 | $CH_3-CH_2-$ | O | 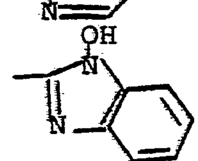 |
| 41 | $CH_3-CH_2-$ | O | 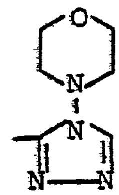 |
| 42 | $CH_3-CH_2-$ | O | 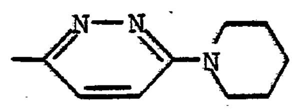 |
| 43 | $CH_3-CH_2-$ | O | 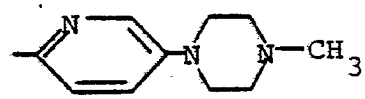 |
| 44 | $CH_3-CH_2-$ | O | 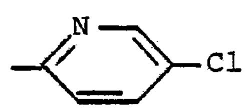 |
| 45 | $CH_3-CH_2-$ | O | 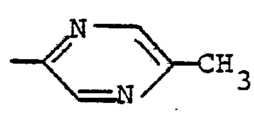 |
| 46 | $CH_3-CH_2-$ | O | 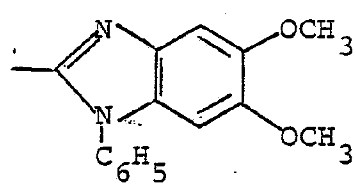 |

Continued.....

| Compound No. | R | n | 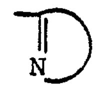 |
| --- | --- | --- | --- |
| 47 | $CH_3-CH_2-$ | 0 | 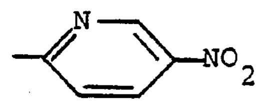 |
| 48 | $CH_3-CH_2-$ | 0 | 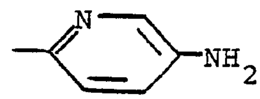 |
| 49 | $CH_3-CH_2-$ | 0 | 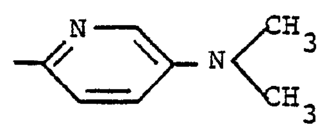 |
| 50 | $CH_3-CH_2-$ | 0 | 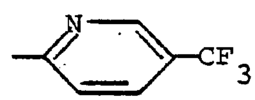 |
| 51 | $CH_3-CH_2-$ | 0 | 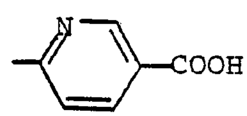 |
| 52 | $CH_3-CH_2-$ | 0 | 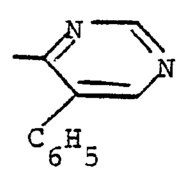 |
| 53 | $CH_3-CH_2-$ | 0 | 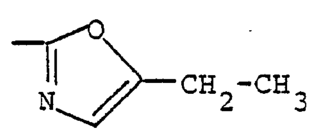 |

Continued.....

| Compound No. | R | n | 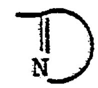 |
| --- | --- | --- | --- |
| 54 | $CH_3CH_2$- | 0 | 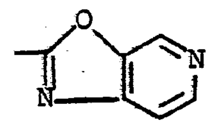 |
| 55 | $CH_3$-$CH_2$- | 0 | 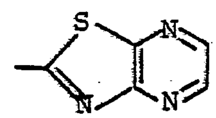 |
| 56 | $CH_3$-$CH_2$- | 0 | 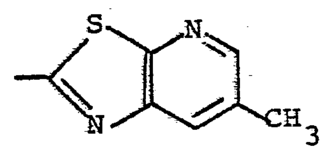 |
| 57 | $CH_3$-$CH_2$- | 0 | 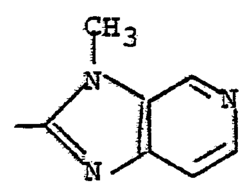 |
| 58 | $CH_3$-$CH_2$- | 0 | 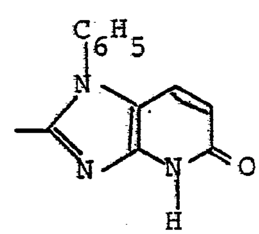 |
| 59 | $CH_3$-$CH_2$- | 0 | 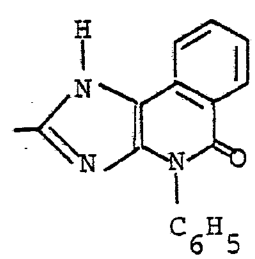 |

Continued.....

| Compound No. | R | n | 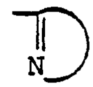 |
|---|---|---|---|
| 60 | $CH_3-CH_2-$ | 0 | 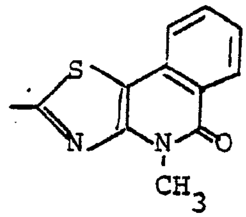 |
| 61 | $CH_3-CH_2-$ | 0 | 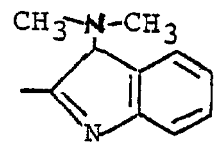 |
| 62 | $CH_3-CH_2-$ | 0 | 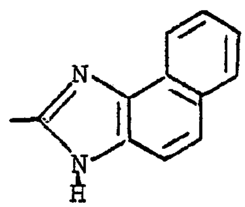 |
| 63 | $CH_3-CH_2-$ | 0 | 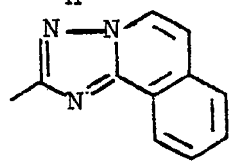 |
| 64 | $CH_3-CH_2-$ | 0 | 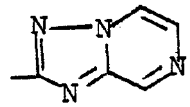 |
| 65 | $CH_3-CH_2-$ | 0 | 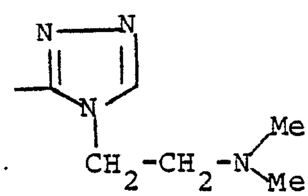 |
| 66 | $CH_3-CH_2-$ | 0 | 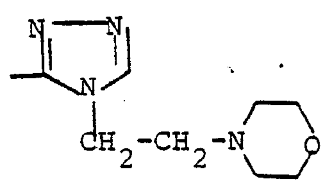 |
| 67 | $CH_3-CH_2-$ | 0 | 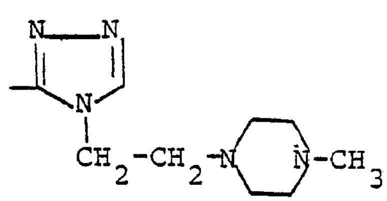 |

Continued.....

| Compound No. | R | n | 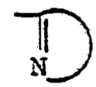 |
|---|---|---|---|
| 68 | $CH_3-CH_2-$ | O | 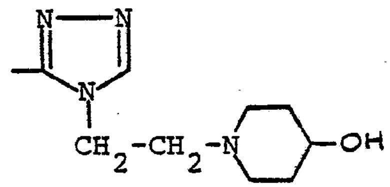 |
| 69 | $CH_3-CH_2-$ | O | 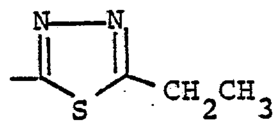 |
| 70 | $CH_3-CH_2-$ | O | 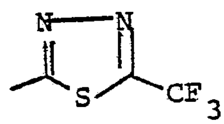 |
| 71 | $CH_3-CH_2-$ | O | 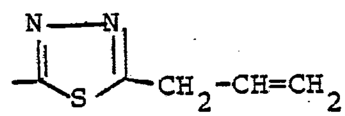 |
| 72 | $CH_3-CH_2-$ | O | 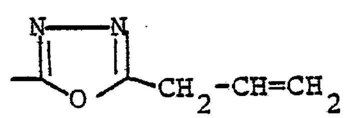 |
| 73 | $CH_3-CH_2-$ | O | 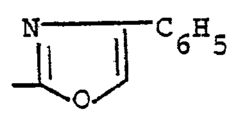 |
| 74 | $CH_3-CH_2-$ | O | 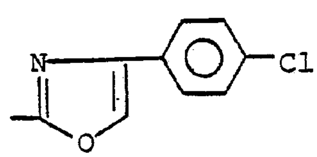 |
| 75 | $CH_3-CH_2-$ | O | 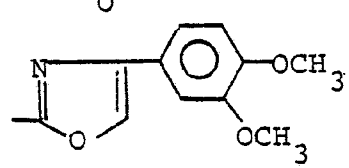 |

Continued.....

| Compound No. | R | n | 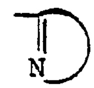 |
|---|---|---|---|
| 76 | $CH_3-CH_2-$ | O | 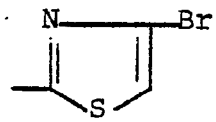 |
| 77 | $CH_3-CH_2-$ | O | 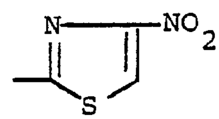 |
| 78 | $CH_3-CH_2-$ | O | 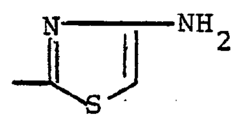 |
| 79 | $CH_3-CH_2-$ | O | 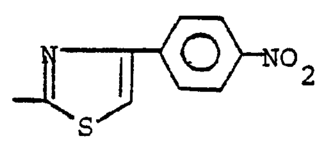 |
| 80 | $CH_3-CH_2-$ | O | 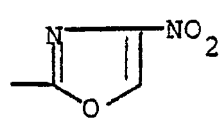 |
| 81 | $CH_3-CH_2-$ | O | 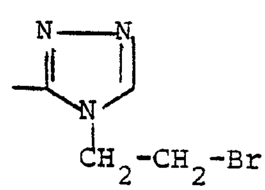 |

CLAIMS

1) A compound of the general formula

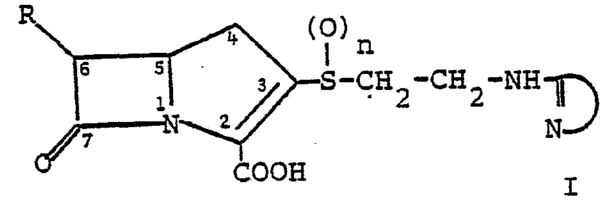

I

wherein R is $(C_1-C_6)$alkyl or $(C_3-C_6)$alkenyl, n is zero or 1 and the stylized group 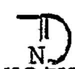 represents a mono-or polycyclic N-containing heterocyclic group, and its pharmaceutically acceptable salts and esters.

2) A compound according to Claim 1 wherein R is ethyl.

3) A compound according to Claim 2 wherein n is zero and the stylized group 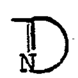 is a monocyclic N-containing heterocyclic group.

4) A process for preparing a compound according to Claim 1 which comprises reacting a β-keto ester of formula II

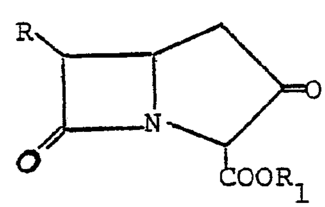

II

wherein R is as defined in Claim 1 and the $-COOR_1$ group represents a cleavable ester or a pharmaceutically acceptable ester group, with diphenyl chlorophosphate whereby

the intermediate enol-phosphate III

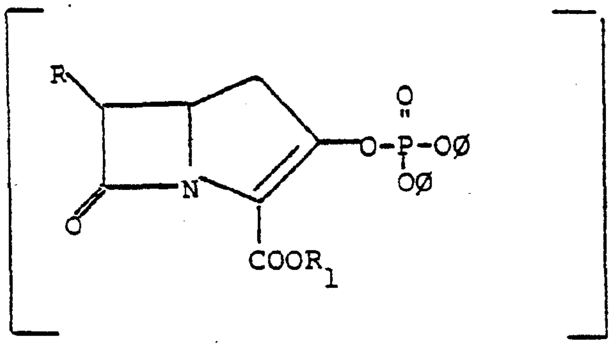

III

is obtained which is reacted with a suitably selected nucleophile of formula

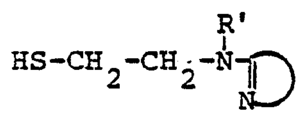

wherein the stylized 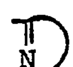 is as defined in Claim 1 and R' is hydrogen or an easily removable protective group of the amino function, to give a compound of formula I wherein R, R' and the group $-COOR_1$ are as defined above, and n is zero, optionally oxidizing the sulfide group to sulfoxide and when a compound of formula I in the form of the free acid or as a corresponding salt thereof is desired, catalytically deprotecting the corresponding cleavable esters thus obtained.

5) A pharmaceutical composition which comprises an antibiotically effective amount of a compound according to Claim 1 and a pharmaceutical carrier therefor.

6) A compound as in any of preceeding Claims 1,2, and 3 wherein the hydrogen atoms at C-5 and C-6 have <u>trans</u> stereochemistry.

European Patent Office

# EUROPEAN SEARCH REPORT

0093915
Application number
EP 83 10 3933

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| Y | EP-A-0 021 082 (MERCK) * Claims * --- | 1,3,5 | C 07 D 487/04<br>C 07 D 519/00<br>A 61 K 31/40 //<br>C 07 D 205/08<br>C 07 D 277/42<br>C 07 D 277/46<br>C 07 D 213/74<br>C 07 D 213/75<br>C 07 D 261/14<br>C 07 D 233/88<br>(C 07 D 519/00<br>C 07 D 498/00<br>C 07 D 487/00 )<br>(C 07 D 519/00<br>C 07 D 513/00<br>C 07 D 487/00 )<br>(C 07 D 487/04<br>C 07 D 209/00<br>-/- |
| Y | EP-A-0 001 628 (MERCK) * Claims * --- | 1,2,5 | |
| A | EP-A-0 044 170 (BEECHAM) * Claims * ----- | 1,5 | |
| | | | **TECHNICAL FIELDS SEARCHED (Int. Cl. 3)** |
| | | | C 07 D 487/00<br>C 07 D 519/00<br>A 61 K 31/00 |

The present search report has been drawn up for all claims

| Place of search | Date of completion of the search | Examiner |
|---|---|---|
| THE HAGUE | 11-07-1983 | CHOULY J. |

**CATEGORY OF CITED DOCUMENTS**

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document

European Patent Office

# EUROPEAN SEARCH REPORT

0093915 Application number

EP 83 10 3933

Page 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| | | | C 07 D 205/00 )<br>(C 07 D 519/00<br>C 07 D 487/00<br>C 07 D 471/00 )<br>(C 07 D 519/00<br>C 07 D 487/00<br>C 07 D 487/00 ) |
| | | | TECHNICAL FIELDS SEARCHED (Int. Cl. 3) |

The present search report has been drawn up for all claims

| Place of search | Date of completion of the search | Examiner |
|---|---|---|
| THE HAGUE | 11-07-1983 | CHOULY J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82